(19) Europäisches Patentamt / European Patent Office / Office européen des brevets

(11) **EP 4 136 262 B1**

(12) **EUROPEAN PATENT SPECIFICATION**

(45) Date of publication and mention of the grant of the patent:
**18.02.2026 Bulletin 2026/08**

(21) Application number: **21717904.3**

(22) Date of filing: **14.04.2021**

(51) International Patent Classification (IPC):
**G16B 20/00** (2019.01)

(52) Cooperative Patent Classification (CPC):
**C12Q 1/6886; G16B 20/00;** C12Q 2600/106;
C12Q 2600/118; C12Q 2600/156; C12Q 2600/158

(86) International application number:
**PCT/EP2021/059695**

(87) International publication number:
**WO 2021/209517 (21.10.2021 Gazette 2021/42)**

(54) **PROGNOSIS METHOD OF ACUTE MYELOID LEUKAEMIA**

PROGNOSEVERFAHREN BEI AKUTER MYELOISCHER LEUKÄMIE

PROCÉDÉ DE PRONOSTIC DE LA LEUCÉMIE MYÉLOÏDE AIGUË

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2020 EP 20315171**

(43) Date of publication of application:
**22.02.2023 Bulletin 2023/08**

(73) Proprietors:
• **Centre national de la recherche scientifique**
  75016 Paris (FR)
• **Centre Hospitalier Universitaire de Montpellier**
  34295 Montpellier Cedex 05 (FR)
• **Université de Montpellier**
  34090 Montpellier (FR)

(72) Inventors:
• **MOREAUX, Jérôme**
  34090 Montpellier (FR)
• **GABELLIER, Ludovic**
  34080 Montpellier (FR)
• **BRET, Caroline**
  34090 Montpellier (FR)

(74) Representative: **Novagraaf Technologies**
**2 rue Sarah Bernhardt**
**CS90017**
**92665 Asnières-sur-Seine Cedex (FR)**

(56) References cited:
WO-A1-2013/138237    WO-A1-2014/044848
WO-A1-2016/012630

• CAROLINE BRET ET AL: "DNA repair in diffuse large B-cell lymphoma: a molecular portrait", BRITISH JOURNAL OF HAEMATOLOGY, vol. 169, 1 January 2015 (2015-01-01), pages 286 - 304, XP055733493
• CAROLINE BRET: "Supplementary Table S2: Identification of DNA repair genes whose expression is associated with a prognostic value in DLBCL patients.", 1 January 2015 (2015-01-01), XP055733495, Retrieved from the Internet <URL:https://onlinelibrary.wiley.com/action/downloadSupplement?doi=10.1111%2Fbjh.13206&file=bjh13206-sup-0006-TableS2.docx> [retrieved on 20200923]
• TARA L. LIN ET AL: "Prognostically Important Molecular Markers in Cytogenetically Normal Acute Myeloid Leukemia", AMERICAN JOURNAL OF MEDICAL SCIENCES, vol. 341, no. 5, 1 May 2011 (2011-05-01), USA, pages 404 - 408, XP055732638, ISSN: 0002-9629, DOI: 10.1097/MAJ.0b013e318201109d

Remarks:
The complete document including Reference Table(s) and the Sequence Listing(s) can be downloaded from the EPO website

**Description**

The invention relates to a method for the prognosis of the outcome of an individual afflicted by an acute myeloid leukaemia (AML).

Acute myeloid leukemia (AML) is a frequent type of adult leukemia. When analyzed with conventional cytogenetics, about 40-50% of AML exhibit no chromosomal abnormalities, and are defined as "cytogenetically normal AML" (CN-AML). Recurrent mutated genes in CN-AML were identified, such as *NPM1,* signal transduction genes (*FLT3*) or myeloid transcription factor genes (*CEBPA, RUNX1*). Based on presence, absence and allelic ratio of these mutations, CN-AML may be classified in favorable, intermediate or adverse prognosis.

Hence, despite improvement in prognosis classification based on the identification of gene mutations such as *NPM1, FLT3* or *CEBPA,* outcomes in CN-AML remain heterogeneous, underlying the wide diversity of this AML subset.

Yet, a wide diversity of gene mutations occurring in CN-AML were revealed by deep sequencing techniques, such as mutations of DNA modification, cohesin or tumor-suppressor genes, suggesting the wide heterogeneity of molecular mechanisms involved in leukemogenesis.

International Application WO 2016/012630 discloses marker sets and scores which are used to predict diffuse large B-cell lymphoma (DLBCL) response to DNA repair pathway inhibitors.

International Application WO 2013/138237 discloses a method to predict survival of acute myeloid leukemia (AML) patients by analyzing the presence of cytogenetic abnormalities and a mutation in at least one of 19 specific genes, and relates to the selection of a suitable treatment.

Bret et al. (British Journal of Haematology, 169 : 286-304, 2015) discloses marker sets and scores for a prognosis of diffuse large B-cell lymphoma (DLBCL).

Hence, alternative methods are still required.

The invention intends to obviate this lack in the art.

On object of the invention is to provide a new efficient prognosis method of acute myeloid leukaemia.

Another object of the invention is to provide a new efficient therapy for treating patients afflicted by AML having a poor outcome.

Thus, the invention relates to a method for the, preferably *in vitro,* prognosis of the outcome of an individual afflicted by an acute myeloid leukaemia with no chromosomal abnormalities and treated by at least one anti-AML drug, said method comprising the following steps:

a) measuring, in a biological sample from said individual, the expression level of all the 23 genes consisting of the nucleic acid sequences as set forth in SEQ ID NO:1 to SEQ ID NO:23;

b) calculating J scores according to the following formula

$$Score_{PATHJ} = \sum_{i=k1}^{kn} \beta i \times Ci$$

wherein βi represents the regression β coefficient reference value for the gene of nucleic acid sequence SEQ ID NO:i,

wherein Ci = 1 if the expression level of the gene of nucleic acid sequence SEQ ID NO:i is higher than an expression level of reference ELR$_i$; or Ci = -1 if the expression level of the gene of nucleic acid sequence SEQ ID NO:i is lower than or equal to ELR$_i$,

wherein J is an integer from 1 to 5 defining five scores Score$_{PATH1}$ to Score$_{PATH5}$, wherein Score$_{PATH1}$ is calculated according to the expression level of genes of nucleic acid sequences SEQ ID NO:A1 to SEQ ID NO:A4 and wherein k is A and n is 4,

Score$_{PATH2}$ is calculated according to the expression level of genes of nucleic acid sequences SEQ ID NO:B1 to SEQ ID NO:B6 and wherein k is B and n is 6,

Score$_{PATH3}$ is calculated according to the expression level of genes of nucleic acid sequences SEQ ID NO:C1 to SEQ ID NO:C6 and wherein k is C and n is 6,

Score$_{PATH4}$ is calculated according to the expression level of genes of nucleic acid sequences SEQ ID NO:D1 to SEQ ID NO:D3 and wherein k is D and n is 3, and

Score$_{PATH5}$ is calculated according to the expression level of genes of nucleic acid sequences SEQ ID NO:E1 to SEQ ID NO:E8 and k is E and n is 8;

c) prognosing that:

i. if at least one of the scores Score$_{PATHJ}$ is higher than its respective reference value Ref$_J$, then the individual is likely to have a bad outcome with a one-year survival (OYS) below 40%,

ii. if all the scores Score$_{PATH1}$ to Score$_{PATH5}$ are lower than or equal to their respective reference value Ref$_1$ to Ref$_5$, then the individual is likely to have a good outcome with an OYS higher than 50%,

iii. if the score Score$_{PATH1}$ is higher than its respective reference value Ref$_1$, then the individual is likely to have a bad outcome with an OYS below 20%,

iv. if the score Score$_{PATH1}$ is lower than or equal to its respective reference value Ref$_1$, then the individual is likely to have a good outcome with an OYS higher than 55%,

v. if the score Score$_{PATH2}$ is higher than its respective reference value Ref$_2$, then the individual is likely to have a bad outcome with an OYS below 25%,

vi. if the score Score$_{PATH2}$ is lower than or equal to its respective reference value Ref$_2$, then the individual is likely to have a good outcome with an OYS higher than 60%,

vii. if the score Score$_{PATH3}$ is higher than its respective reference value Ref$_3$, then the individual is likely to have a bad outcome with an OYS below 30%,

viii. if the score Score$_{PATH3}$ is lower than or equal to its respective reference value Ref$_3$, then the individual is likely to have a good outcome with an OYS higher than 70%,

ix. if the score Score$_{PATH4}$ is higher than its respective reference value Ref$_4$, then the individual is likely to have a bad outcome with an OYS below 10%,

x. if the score Score$_{PATH4}$ is lower than or equal to its respective reference value Ref$_4$, then the individual is likely to have a good outcome with an OYS higher than 50%,

xi. if the score Score$_{PATH5}$ is higher than a its respective reference value Ref$_5$, then the individual is likely to have a bad outcome with an OYS below 40%,

xii. if the score Score$_{PATH5}$ is lower than or equal to its respective reference value Ref$_5$, then the individual is likely to have a good outcome with an OYS higher than 80%,

wherein the reference value for Score$_{PATH1}$ is Ref$_1$ and equals to 0.022276,

the reference value for Score$_{PATH2}$ is Ref$_2$ and equals to -0.57621,

the reference value for Score$_{PATH3}$ is Ref$_3$ and equals to -0.26745

the reference value for Score$_{PATH4}$ is Ref$_4$ and equals to 0.012234, and

the reference value for Score$_{PATH5}$ is Ref$_5$ and equals to -1.1213.

The inventors have identified a set of 23 genes and/or proteins, which are differentially expressed in individuals having an AML as compared to healthy individuals and determined as relevant for their prognosis. These 23 genes, which have never been associated together with AML, belong to 5 different DNA repair pathways, namely Base Excision Repair (BER) pathway, Fanconi (FANC) pathway, Homologous Recombination Repair (HRR) pathway, Mismatch Repair (MMR) pathway and Nucleotide Excision Repair (NER) pathway.

In the invention, the term "individual" refers to a mammal individual, preferably a human individual.

In the invention, "acute myeloid leukaemia" or "AML" refers to a cancer of the myeloid lineage of blood cells, characterized by the rapid growth of abnormal cells that build up in the bone marrow and blood and interfere with normal blood cells. Particularly in the invention, the AML is associated with no chromosomal abnormalities, also defined as "cytogenetically normal AML" (CN-AML), well known in the art. As an example, this phenotype can be determined by examining the appearance of the malignant cells with light microscopy or fluorescence microscopy to characterize any underlying chromosomal abnormalities.

In the invention, "anti-AML drug" refers to any drug that are used to treat acute myeloid leukaemia. Anti-AML drugs can be selected in the group comprising, but are not limited to, daunorubicin, idarubicin, cytarabine, midostaurin, cladribine, gemtuzumab ozogamicin, and a combination thereof.

The term "outcome" refers to the survival, the relapse or the death of the individual. The outcome may relate to disease-free survival (DFS), event free survival (EFS) or overall survival (OS), as defined within the state of the art. Illustratively, a "bad outcome" may refer to a disease relapse or death of the individual. Oppositely, a "good outcome" may refer to survival of the individual, with or without relapse episode.

Step a)

In the invention, a "biological sample" refers to a biological sample obtained, reached, collected or isolated from an individual, *in vivo* or *in situ*. Such samples may be, but not limited to, organs, tissues, fractions and cells isolated from an individual. For example, suitable biological samples include but are not limited to a cell culture, a cell line, a tissue biopsy such as a bone marrow aspirate, a biological fluid such as a blood, pleural effusion or a serum sample, and the like. An advantageous biological sample includes but is not limited to a blood sample, a tissue biopsy, including a bone marrow aspirate. The biological sample as defined in the invention may be a crude sample, or may be purified to various degrees prior to storage, processing, or measurement.

The expression level of the genes is measured by well-known protocol in the art. These methods are for instance, DNA-CHIPs containing probesets of said 23 genes, so that an expression level can be determined for each of said 23 genes. Other methods can be used, such that quantitative PCR strategy by using specific couples of primers for each of said 23 genes, with either a specific Taqman probe for each of said 23 genes, or SYBR® compounds.

Advantageously, the expression level can be evaluated by measuring the expression level of mRNA for each of the genes of interest. This measurement may be carried out by using the well-known techniques available in the art. In this case, mRNA may be extracted, for example using lytic enzymes or chemical solutions or extracted by commercially available nucleic-acid-binding resins following the manufacturer's instructions. Extracted mRNA may be subsequently detected by hybridization, such as Northern blot, and/or amplification, such as quantitative or semi-quantitative RT-PCR. Other methods of amplification include ligase chain reaction (LCR), transcription-mediated amplification (TMA), strand displacement amplification (SDA) and nucleic acid sequence-based amplification (NASBA).

Advantageously, the level of mRNA expression for each of the genes of interest may be measured by the mean of quantification of the cDNA synthesized from said mRNA, as a template, by one reverse transcriptase. Methods for determining the quantity of mRNA by microarrays or by RNA sequencing may also be used.

In certain embodiments, complexes between the double-stranded nucleic acids resulting from amplification and fluorescent SYBR® molecules may be obtained and then the fluorescence signal generated by the SYBR® molecules complexed with the said amplified nucleic acids may be measured.

The determination of the expression level of said 23 genes could be to carry out by a northern blot analysis, but due to the low efficiency of such a method, the skilled person will prefer the quantitative methods to obtain a more precise expression level of said 23 genes.

The set of 23 genes of the invention with the corresponding probe set and CDS (or one of the CDS if the gene expression different variants) are represented in the following table:

| Probe set | Gene symbole | CDS SEQ ID |
|---|---|---|
| 210027_s_at | APEX1 | SEQ ID NO :1 |
| 209731_at | NTHL1 | SEQ ID NO :2 |
| 202330_s_at | UNG | SEQ ID NO :3 |
| 203655_at | XRCC1 | SEQ ID NO :4 |
| 209902_at | ATR | SEQ ID NO :5 |
| 214727_at | BRCA2 | SEQ ID NO :6 |
| 203719_at | ERCC1 | SEQ ID NO :7 |

EP 4 136 262 B1

(continued)

| Probe set | Gene symbole | CDS SEQ ID |
|---|---|---|
| 203678_at | FAN1 | SEQ ID NO :8 |
| 221206_at | PMS2 /// PMS2CL | SEQ ID NO :9 |
| 219317_at | POLI | SEQ ID NO :10 |
| 205395_s_at | MRE11 | SEQ ID NO : 11 |
| 205647_at | RAD52 | SEQ ID NO :12 |
| 206092_x_at | RTEL1 | SEQ ID NO :13 |
| 212275_s_at | SRCAP | SEQ ID NO :14 |
| 207598_x_at | XRCC2 | SEQ ID NO :15 |
| 205887_x_at | MSH3 | SEQ ID NO :16 |
| 1053_at | RFC2 | SEQ ID NO :17 |
| 201405_s_at | COPS6 | SEQ ID NO :18 |
| 213579_s_at | EP300 | SEQ ID NO :19 |
| 205162_at | ERCC8 | SEQ ID NO :20 |
| 223758_s_at | GTF2H2 | SEQ ID NO :21 |
| 201046_s_at | RAD23A | SEQ ID NO :22 |
| 205672_at | XPA | SEQ ID NO :23 |

In this table, it has to be noted that when several CDS are given for a gene, the SEQ ID NO corresponds to the first one. Nevertheless, the given prob set for the said gene is able to recognize every CDS of the said gene, i.e. including the ones not mentioned therein.

Step b)

In the above method, when the expression level of said 23 genes was measured, step b) is carried out. Step b) consist first in calculating J scores according to the following formula

$$Score_{PATHJ} = \sum_{i=k1}^{kn} \beta i \times Ci$$

J is an integer ranging from 1 to 5. Accordingly, in this formula $Score_{PATHJ}$ defines 5 scores namely $Score_{PATH1}$, $Score_{PATH2}$, $Score_{PATH3}$, $Score_{PATH4}$ and $Score_{PATH5}$, and at least one this score is calculated within step b).
As abovementioned, the set of 23 genes of the invention are allocated to 5 groups of genes, each one corresponding to a particular DNA repair pathway group of genes: BER pathway, FANC pathway, HRR pathway, MMR pathway and NER pathway.
As represented in this Table 1, one gene can be allocated to several of the 5 groups of genes.
By analogy, each $Score_{PATHJ}$ is calculated with the expression level of the genes attributed to one of the 5 groups of genes, as represented in Table 1 below.
Each gene of each of the 5 groups of genes is assigned with a substitute SEQ ID NO according to the DNA repair pathway group they belong to (letter A for the BER pathway group, letter B for the FANC pathway group, letter C for the HRR pathway group, letter D for the MMR pathway group and letter E for the NER pathway group), as represented in the below Table 1:

Table 1

| DNA repair pathway Group | Score$_{PATH J}$ | Gene symbol | CDS SEQ ID | Substitute SEQ ID NO | β coefficient | Expression level of reference (ELR) |
|---|---|---|---|---|---|---|
| BER pathway | Score$_{PATH1}$ | APEX1 | SEQ ID NO :1 | SEQ ID NO :A1 | 0.20411998 | 5148.72678 |
| | | NTHL1 | SEQ ID NO :2 | SEQ ID NO :A2 | 0.2787536 | 235.568039 |
| | | UNG | SEQ ID NO :3 | SEQ ID NO :A3 | 0.30103 | 803.414116 |
| | | XRCC1 | SEQ ID NO :4 | SEQ ID NO :A4 | 0.20411998 | 433.5336 |
| FANC pathway | Score$_{PATH2}$ | ATR | SEQ ID NO :5 | SEQ ID NO :B1 | 0.25527251 | 261.379104 |
| | | BRCA2 | SEQ ID NO :6 | SEQ ID NO :B2 | -0.236572 | 106.891254 |
| | | ERCC1 | SEQ ID NO :7 | SEQ ID NO :B3 | 0.2787536 | 1269.46068 |
| | | FAN1 | SEQ ID NO :8 | SEQ ID NO :B4 | 0.25527251 | 689.783591 |
| | | PMS2 /// PMS2CL | SEQ ID NO :9 | SEQ ID NO :B5 | 0.25527251 | 1024 |
| | | POLI | SEQ ID NO :10 | SEQ ID NO :B6 | 0.2787536 | 224.411065 |
| HRR pathway | Score$_{PATH3}$ | BRCA2 | SEQ ID NO :6 | SEQ ID NO :C1 | -0.236572 | 106.891254 |
| | | MRE11 | SEQ ID NO :11 | SEQ ID NO :C2 | 0.25527251 | 699.412611 |
| | | RAD52 | SEQ ID NO :12 | SEQ ID NO :C3 | 0.2787536 | 135.298309 |
| | | RTEL1 | SEQ ID NO :13 | SEQ ID NO :C4 | 0.39794001 | 407.31468 |
| | | SRCAP | SEQ ID NO :14 | SEQ ID NO :C5 | -0.2218487 | 484.381515 |
| | | XRCC2 | SEQ ID NO :15 | SEQ ID NO :C6 | 0.23044892 | 274.374006 |
| MMR pathway | Score$_{PATH4}$ | MSH3 | SEQ ID NO :16 | SEQ ID NO :D1 | 0.44715803 | 150.12287 |
| | | PMS2 /// PMS2CL | SEQ ID NO :9 | SEQ ID NO :D2 | 0.25527251 | 1024 |
| | | RFC2 | SEQ ID NO :17 | SEQ ID NO :D3 | 0.20411998 | 367.092543 |

(continued)

| DNA repair pathway Group | Score$_{PATH\ J}$ | Gene symbol | CDS SEQ ID | Substitute SEQ ID NO | β coefficient | Expression level of reference (ELR) |
|---|---|---|---|---|---|---|
| NER pathway | Score$_{PATH5}$ | COPS6 | SEQ ID NO :18 | SEQ ID NO :E1 | 0.23044892 | 1770.57225 |
| | | EP300 | SEQ ID NO :19 | SEQ ID NO :E2 | -0.229148 | 634.730342 |
| | | ERCC1 | SEQ ID NO :7 | SEQ ID NO :E3 | 0.2787536 | 1269.46068 |
| | | ERCC8 | SEQ ID NO :20 | SEQ ID NO :E4 | 0.17609126 | 504.951145 |
| | | GTF2H2 | SEQ ID NO :21 | SEQ ID NO :E5 | 0.17609126 | 181.019336 |
| | | RAD23A | SEQ ID NO :22 | SEQ ID NO :E6 | -0.2757241 | 2646.7386 |
| | | | | | | |
| | | XPA | SEQ ID NO :23 | SEQ ID NO :E7 | 0.25527251 | 526.394279 |
| | | XRCC1 | SEQ ID NO :4 | SEQ ID NO :E8 | 0.20411998 | 433.5336 |

Score$_{PATH1}$ is assigned to BER pathway group and is calculated according to the expression of genes of nucleic acid sequences SEQ ID NO:A1 to SEQ ID NO:A4. Accordingly, for the calculation of Score$_{PATH1}$ with the abovementioned formula, k is A and n is 4 such that i varies from A1 to A4. The formula of Score$_{PATH1}$ is therefore as follows:

$$Score_{PATH1} = \sum_{i=A1}^{A4} \beta i \times Ci$$

In this group, for each gene, a high expression level, i.e. an expression level higher than their respective ELR value, is associated with a bad prognosis.

Score$_{PATH2}$ is assigned to FANC pathway group and is calculated according to the expression of genes of nucleic acid sequences SEQ ID NO:B1 to SEQ ID NO:B6. Accordingly, for the calculation of Score$_{PATH2}$ with the abovementioned formula, k is B and n is 6 such that i varies from B1 to B6. The formula of Score$_{PATH2}$ is therefore as follows:

$$Score_{PATH2} = \sum_{i=B1}^{B6} \beta i \times Ci$$

In this group, for the gene of nucleic acid sequence SEQ ID NO:B2, a high expression level is associated with a good prognosis, while for the others genes, a high expression level is associated with a bad prognosis.

Score$_{PATH3}$ is assigned to HRR pathway group and is calculated according to the expression of genes of nucleic acid sequences SEQ ID NO:C1 to SEQ ID NO:C6. Accordingly, for the calculation of Score$_{PATH3}$ with the abovementioned formula, k is C and n is 6 such that i varies from C1 to C6. The formula of Score$_{PATH3}$ is therefore as follows:

$$Score_{PATH3} = \sum_{i=C1}^{C6} \beta i \times Ci$$

In this group, for the genes of nucleic acid sequences SEQ ID NO:C1 and SEQ ID NO:C5, a high expression level is associated with a good prognosis, while for the others genes, a high expression level is associated with a bad prognosis.

Score$_{PATH4}$ is assigned to MMR pathway group and is calculated according to the expression of genes of nucleic acid sequences SEQ ID NO:D1 to SEQ ID NO:D3. Accordingly, for the calculation of Score$_{PATH4}$ with the abovementioned formula, k is D and n is 3 such that i varies from D1 to D3. The formula of Score$_{PATH4}$ is therefore as follows:

$$Score_{PATH4} = \sum_{i=D1}^{D3} \beta i \times Ci$$

In this group, for each gene, a high expression level is associated with a bad prognosis.

Score$_{PATH5}$ is assigned to NER pathway group and is calculated according to the expression of genes of nucleic acid sequences SEQ ID NO:E1 to SEQ ID NO:E8. Accordingly, for the calculation of Score$_{PATH5}$ with the abovementioned formula, k is E and n is 8 such that i varies from D1 to D8. The formula of Score$_{PATH5}$ is therefore as follows:

$$Score_{PATH5} = \sum_{i=E1}^{E8} \beta i \times Ci$$

In this group, for the genes of nucleic acid sequences SEQ ID NO:E2 and SEQ ID NO:E6, a high expression level is associated with a good prognosis, while for the others genes, a high expression level is associated with a bad prognosis.

In the abovementioned formula, $\beta i$ represents the regression $\beta$ coefficient reference value for the gene of nucleic acid sequence SEQ ID NO:i, wherein "i" is as defined above for each DNA repair pathway group.

The regression $\beta$ coefficient reference values may be easily determined by the skilled man in the art for each gene of nucleic acid sequence SEQ ID NO:i using a Cox model. The Cox model is based on a modelling approach to the analysis of survival data. The purpose of the model is to simultaneously explore the effects of several variables on survival. The Cox model is a well-recognised statistical technique for analysing survival data. When it is used to analyse the survival of patients in a clinical trial, the model allows us to isolate the effects of treatment from the effects of other variables. The logrank test cannot be used to explore (and adjust for) the effects of several variables, such as age and disease duration, known to affect survival. Adjustment for variables that are known to affect survival may improve the precision with which the inventors can estimate the treatment effect. The regression method introduced by Cox is used to investigate several variables at a time. It is also known as proportional hazards regression analysis. Briefly, the procedure models or regresses the survival times (or more specifically, the so-called hazard function) on the explanatory variables. The hazard function is the probability that an individual will experience an event (for example, death) within a small-time interval, given that the individual has survived up to the beginning of the interval. It can therefore be interpreted as the risk of dying at time $t$. The quantity h0 ($t$) is the baseline or underlying hazard function and corresponds to the probability of dying (or reaching an event) when all the explanatory variables are zero. The baseline hazard function is analogous to the intercept in ordinary regression (since exp0= 1). The regression coefficient $\beta$ gives the proportional change that can be expected in the hazard, related to changes in the explanatory variables. The coefficient $\beta$ is estimated by a statistical method called maximum likelihood. In survival analysis, the hazard ratio (HR) (Hazard Ratio= exp($\beta$)) is the ratio of the hazard rates corresponding to the conditions described by two sets of explanatory variables. For example, in a drug study, the treated population may die at twice the rate per unit time as the control population. The hazard ratio would be 2, indicating higher hazard of death from the treatment.

In one embodiment, the regression $\beta$ coefficient reference values are described in Table 1.

In the abovementioned formula, Ci = 1 if the expression level of the gene of nucleic acid sequence SEQ ID NO:i is higher than an expression level of reference ELR$_i$ or Ci = -1 if the expression level of the gene of nucleic acid sequence SEQ ID NO:i is lower than or equal to ELR$_i$, wherein "i" is as defined above for each DNA repair pathway group.

Expression level of reference ELR$_i$ may consist of *"cut-off"* values. A cut-off value is a value of expression of a gene that allows to separate the individuals according to their outcome (good or bad) for a given gene. If the measured expression value of the gene of an individual is higher than the cut-off value, the individual has a good outcome and *vice-versa.* The cut-off values may be obtained using Maxstat algorithm.

For example, each reference cut-off value ELRi for each gene may be determined by carrying out a method comprising the steps of:

a) providing a collection of samples from patients suffering from acute myeloid leukemia;
b) determining the expression level of the relevant gene for each sample contained in the collection provided at step a);
c) ranking the samples according to said expression level;
d) classifying said samples in pairs of subsets of increasing, respectively decreasing, number of members ranked according to their expression level;

e) providing, for each sample provided at step a), information relating to the actual clinical outcome for the corresponding cancer patient (i.e. the duration of the disease-free survival (DFS), or the event free survival (EFS), or the overall survival (OS) or both);

f) for each pair of subsets of tumour tissue samples, obtaining a Kaplan Meier percentage of survival curve;

g) for each pair of subsets of tumour tissue samples calculating the statistical significance (p value) between both subsets;

h) selecting as reference value ELR for the expression level, the value of expression level for which the p value is the smallest.

For example, the expression level of a gene has been assessed for 100 samples of 100 patients. The 100 samples are ranked according to the expression level of the gene. Sample 1 has the highest expression level and sample 100 has the lowest expression level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves are prepared for each of the 99 groups of two subsets. Also, for each of the 99 groups, the p value between both subsets was calculated. The reference value ELRi is then selected such as the discrimination based on the criterion of the minimum p value is the strongest. In other terms, the expression level corresponding to the boundary between both subsets for which the p value is minimum is considered as the reference value. It should be noted that according to the experiments made by the inventors, the reference value ELRi is not necessarily the median value of expression levels.

In one embodiment, the $ELR_i$ are described in Table 1.

Step c)

When at least one of the scores $Score_{PATHJ}$ is calculated as mentioned above, it is proposed to classify the individual from which the biological sample has been recovered either as likely to have a good outcome or a bad outcome and to attribute a one-year-survival (OYS).

By "one-year-survival" or "OYS", it is meant in the invention the percentage of chance for the individual to survive at one year from the day the expression level of the 23 genes were carried out, i.e. when step a is carried out.

In that purpose, the calculated $Score_{PATHJ}$ are compared with a respective reference value $Ref_J$. The said reference values $Ref_J$ are the following cut-off values:

- the reference value for $Score_{PATH1}$ is $Ref_1$ and equals to 0.022276,
- the reference value for $Score_{PATH2}$ is $Ref_2$ and equals to -0.57621,
- the reference value for $Score_{PATH3}$ is $Ref_3$ and equals to -0.26745
- the reference value for $Score_{PATH4}$ is $Ref_4$ and equals to 0.012234, and
- the reference value for $Score_{PATH5}$ is Ref5 and equals to -1.1213.

The reference values $Ref_J$ may be determined as above presented for the ELRi value.

From the said comparisons, it can be made the below prognosis.

If at least one of the scores $Score_{PATHJ}$ is higher than a respective reference value $Ref_J$, then the individual is likely to have a bad outcome with a OYS below 40%. By a "OYS below 40%", it is meant in the invention 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% and 0%.

If all the scores $Score_{PATH1}$ to $Score_{PATH5}$ are lower than or equal to their respective reference value $Ref_1$ to $Ref_5$, then the individual is likely to have a good outcome with an OYS higher than 50%. By a "OYS higher than 50%", it is meant in the invention 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 100%.

If the score $Score_{PATH1}$ is higher than a its respective reference value $Ref_1$, then the individual is likely to have a bad outcome with an OYS below 20%. By a "OYS below 20%", it is meant in the invention 20%, 19%, 18%, 17%, 16%, 15%, 14%, 13%, 12%, 11%, 10%, 9%, 8%, 7%, 6%, 5%, 4%, 3%, 2%, 1% and 0%.

If the score $Score_{PATH1}$ is lower than or equal to its respective reference value $Ref_1$, then the individual is likely to have a good outcome with an OYS higher than 55%. By a "OYS higher than 55%", it is meant in the invention 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 100%.

If the score $Score_{PATH2}$ is higher than a its respective reference value $Ref_2$, then the individual is likely to have a bad outcome with an OYS below 25%. By a "OYS below 25%", it is meant in the invention 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% and 0%.

If the score $Score_{PATH2}$ is lower than or equal to its respective reference value $Ref_2$, then the individual is likely to have a good outcome with an OYS higher than 60%. By a "OYS higher than 60%", it is meant in the invention 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 100%.

If the score $Score_{PATH3}$ is higher than a its respective reference value $Ref_3$, then the individual is likely to have a bad outcome with an OYS below 30%. By a "OYS below 30%", it is meant in the invention 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% and 0%.

If the score $Score_{PATH3}$ is lower than or equal to its respective reference value $Ref_3$, then the individual is likely to have a good outcome with an OYS higher than 70%. By a "OYS higher than 70%", it is meant in the invention 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 100%.

If the score $Score_{PATH4}$ is higher than a its respective reference value $Ref_4$, then the individual is likely to have a bad outcome with an OYS below 10%. By a "OYS below 10%", it is meant in the invention 10%, 9%, 8%, 7%, 6%, 5%, 4%, 3%, 2%, 1% and 0%.

If the score $Score_{PATH4}$ is lower than or equal to its respective reference value $Ref_4$, then the individual is likely to have a good outcome with an OYS higher than 50%. By a "OYS higher than 50%", it is meant in the invention 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 100%.

If the score $Score_{PATH5}$ is higher than a its respective reference value $Ref_5$, then the individual is likely to have a bad outcome with an OYS below 40%. By a "OYS below 40%", it is meant in the invention 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% and 0%.

If the score $Score_{PATH5}$ is lower than or equal to its respective reference value $Ref_5$, then the individual is likely to have a good outcome with an OYS higher than 80%. By a "OYS higher than 80%", it is meant in the invention 80%, 81%, 82%, 83%, 84%, 85%, 86%, 87%, 88%, 89%, 90%, 91%, 92%, 93%, 94%, 95%, 96%, 97%, 98%, 99% and 100%.

The inventors discovered that the above prognosis can be refined by combining the results of $Score_{PATH3}$ and $Score_{PATH5}$, that is, by using the expression level of the genes pertaining to HRR pathway group and NER pathway group. The inventors defined three outcoming groups, namely a low risk group, a median risk group and a high-risk group.

In an advantageous embodiment, step c) further comprises the below prognosis:

- if both scores $Score_{PATH3}$ and $Score_{PATH5}$ are lower than their respective reference value $Ref_3$ and $Ref_5$, then the individual belongs to a low risk group with an OYS higher than 80%. By a "OYS higher than 80%", it is meant in the invention 80%, 81%, 82%, 83%, 84%, 85%, 86%, 87%, 88%, 89%, 90%, 91%, 92%, 93%, 94%, 95%, 96%, 97%, 98%, 99% and 100%;

- if one of the scores $Score_{PATH3}$ and $Score_{PATH5}$ is higher than its respective reference value $Ref_3$ or $Ref_5$, and the other one is lower than its respective reference value $Ref_3$ or $Ref_5$, then the individual belongs to a median risk group with an OYS between 60 and 80%. By a "OYS between 60 and 80%" it is meant in the invention 61%, 62%, 63%, 64%, 65%, 66%, 67%, 68%, 69%, 70%, 71%, 72%, 73%, 74%, 75%, 76%, 77%, 78% and 79%; and

- if both $Score_{PATH3}$ and $Score_{PATH5}$ are higher than their respective reference value $Ref_3$ and $Ref_5$, then the individual belongs to a high-risk group with an OYS below 30%. By a "OYS below 30%", it is meant in the invention 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% and 0%.

In another advantageous embodiment, the method further comprises the following steps:

d) determining the mutational status of both the *NPM1* gene and *FLT3* gene, said *NPM1* gene having a nucleic acid sequence as set forth in SEQ ID NO:24 and said *FLT3* gene having a nucleic acid sequence as set forth in SEQ ID NO:25,

wherein if the *NPM1* gene is mutated such that it codes for a NMP1 protein which is delocalized into the cytoplasm, the individual is classified as *NPM1+,*

otherwise the individual is classified as *NPM1-,*

if the *FLT3* gene is mutated such that it codes for a FLT3 protein having an internal tandem duplication in the juxta-transmembrane domain, the individual which is classified as *FLT3*-ITD+,

otherwise the individual is classified as *FLT3*-ITD-;

e) classifying the individual such that

i. the individual belongs to a group A with an OYS higher than 85% if

- the individual is *NPM1+* and *FLT3*-ITD- and belongs to the low risk group or the median risk group according to step c), or

- the individual is classified *NPM1+* and *FLT3*-ITD+ or *NPM1-* and *FLT3*-ITD-, and belongs to the low risk group according to step c)

ii. the individual belongs to a group B with OYS between 40 and 60%, if

- the individual is classified *NPM1+* and *FLT3*-ITD- and belongs to the high-risk group according to step c),

- the individual is classified *NPM1+* and *FLT3*-ITD+ or *NPM1-* and *FLT3*-ITD-, and belongs to the median risk group according to step c), or

- the individual is classified *NPM1-* and *FLT3*-ITD+ and belongs to the low risk group according to step c)

iii. the individual belongs to a group C with an OYS below 30% if

- the individual is classified *NPM1+* and *FLT3*-ITD+ or *NPM1-* and *FLT3*-ITD-, and belongs to the high-risk group according to step c), or

- the individual is classified *NPM1-* and *FLT3*-ITD+ and belongs to the median risk group or the high-risk group according to step c).

The mutational status of both genes *NMP1* and *FLT3* are widely used for prognosing the outcome of an individual afflicted by an AML.

NPM1 protein is associated with nucleolar ribonucleoprotein structures and binds single-stranded and double-stranded nucleic acids, but it binds preferentially G-quadruplex forming nucleic acids. It is involved in the biogenesis of ribosomes and may assist small basic proteins in their transport to the nucleolus. Mutations of *NPM1* gene are known to be associated with good prognosis in CN-AML and well known in the art. Briefly AML carrying an *NPM1* gene mutation causing aberrant cytoplasmic expression of NPM1 accounts for approximately one-third of adult AML. Currently more than 40 different mutations in the *NPM1* gene have been identified. All these variants lead to common changes at the C-terminus of the gene and cause aberrant dislocation of the NPM1 protein into the cytoplasm of AML blast cells.

FLT3 is a cytokine receptor which belongs to the receptor tyrosine kinase class III. This receptor is expressed on the surface of many hematopoietic progenitor cells. Signalling of FLT3 is important for the normal development of haematopoietic stem cells and progenitor cells. Briefly *FLT3* gene is mutated in about 1/3 of AML patients. *FLT3* gene mutations consist of two major types: internal tandem duplication (ITD) mutations of 3-400 base pairs (always in-frame) that map to the juxta-membrane region (23% of AML patients) and point mutations that most frequently involve aspartic acid 835 of the kinase domain (KD) but have also been found less frequently in several other sites (8-12% of AML patients). Because ITD mutations interfere with the negative regulatory function of the juxta-membrane region and KD point mutations most frequently involve the activation loop, both types of mutations result in constitutive activation of FLT3 in the absence of ligand. Mutations of *FLT3* gene are known to drive AML and associated with poor prognosis in CN-AML for FLT3-ITD mutations. The associated mechanisms are well known in the art.

The inventors investigate the interest of combining the above defined outcoming groups (low risk group, median risk group and a high-risk group) with *NPM1* and *FLT3* mutational status. They surprisingly discovered that this combination refines both the outcome of the individual classified in the outcoming groups and the outcome of patients for which the prognosis is established from *NMP1* and *FLT3* mutational status.

The individual is classified according to the outcoming groups he belongs to (0 point for low risk group; 1 point for median risk group and 2 for high-risk group), and his *NPM1* and *FLT3* mutational status (0 point if *NPM1+* and *FLT3*-ITD-; 2 points if *FLT3*-ITD+ and *NPM1-;* 1 point in other situations). The sum of the prognostic information allows to attribute the individual into three new prognosis groups: group A for an individual with 0 or 1 point, group B for an individual with 2 points and group C for an individual with 3 or 4 points. (see Table 2 below).

| | | Outcoming groups | | |
|---|---|---|---|---|
| | | Group I (*0 point*) | Group II (*1 point*) | **Group III** (*2 points*) |
| **NPM1 and FLT3 mutational status** | *NPM1+* and *FLT3*-ITD-(*0 point*) | **Group A** (*0 point*) | **Group A** (1 *point*) | **Group B** (*2 points*) |
| | *NPM1+* and *FLT3*-ITD+ *or NPM1-* and *FLT3*-ITD-(*1 point*) | **Group A** (*1 point*) | **Group B** (2 *points*) | **Group C** (3 *points*) |
| | *NPM1-* and *FLT3*-ITD+ (*2 points*) | **Group B** (*2 points*) | **Group C** (*3 points*) | **Group C** (4 *points*) |

In one embodiment, the biological sample is selected in the group comprising a cell culture, a cell line, a tissue biopsy such as a bone marrow aspirate, a biological fluid such as a blood, pleural effusion and a serum sample.

In one embodiment, wherein the at least one drug used for treating acute myeloid leukaemia is chosen from daunorubicin, idarubicin, cytarabine midostaurin, cladribine, gemtuzumab ozogamicin and a combination thereof.

A method for, with preference *in vitro,* determining if an individual afflicted by an acute myeloid leukaemia with no chromosomal abnormalities and treated by at least one drug used for treating acute myeloid leukaemia will respond to a DNA repair pathway inhibitor is also disclosed which is not part of the present invention, said method comprising the following steps:

a) measuring in a biological sample from said individual, the expression level of the 23 genes consisting of the nucleic acid sequences SEQ ID NO:1 to SEQ ID NO:23;

b) calculating J scores according to the following formula

$$Score_{PATHJ} = \sum_{i=k1}^{kn} \beta i \times Ci$$

wherein $\beta i$ represents the regression $\beta$ coefficient reference value for the gene of nucleic acid sequence SEQ ID NO:i,

wherein $Ci = 1$ if the expression level of the gene of nucleic acid sequence SEQ ID NO:i is higher than an expression level of reference $ELR_i$ or $Ci = -1$ if the expression level of the gene of nucleic acid sequence SEQ ID NO:i is lower than or equal to $ELR_i$,

wherein J is an integer from 1 to 5 defining five scores $Score_{PATH1}$ to $Score_{PATH5}$,

wherein $Score_{PATH1}$ is calculated according to the expression level of genes of nucleic acid sequences SEQ ID NO:A1 to SEQ ID NO:A4 and wherein k is A and n is 4,

$Score_{PATH2}$ is calculated according to the expression level of genes of nucleic acid sequences SEQ ID NO:B1 to SEQ ID NO:B6 and wherein k is B and is 6,

$Score_{PATH3}$ is calculated according to the expression level of genes of nucleic acid sequences SEQ ID NO:C1 to SEQ ID NO:C6 and wherein k is C and n is 6,

$Score_{PATH4}$ is calculated according to the expression level of genes of nucleic acid sequences SEQ ID NO:D1 to SEQ ID NO:D3 and wherein k is D and n is 3, and

$Score_{PATH5}$ is calculated according to the expression level of genes of nucleic acid sequences SEQ ID NO:E1 to SEQ ID NO:E8 and wherein k is E and n is 8;

c) classifying the individual such that

i. if $Score_{PATH1}$ is higher than the reference value 0.022276, then the individual is likely to respond to a Base Excision Repair pathway inhibitor,

ii. if $Score_{PATH2}$ is higher than the reference value -0.57621, then the individual is likely to respond to a Fanconi pathway inhibitor,

iii. if $Score_{PATH3}$ is higher than the reference value -0.26745, then the individual is likely to respond to a Homologous Recombination Repair pathway inhibitor,

iv. if $Score_{PATH4}$ is higher than the reference value 0.012234, then the individual is likely to respond to a Mismatch Repair pathway inhibitor,

v. if $Score_{PATH5}$ is higher than the reference value -1.1213, then the individual is likely to respond to a Nucleotide Excision Repair pathway inhibitor,

vi. if a score from scores $Score_{PATH1}$ to $Score_{PATH5}$ is lower than the respective reference value, then the individual is likely to not respond to the respective DNA repair pathway inhibitor.

Steps a) and b) of this method are the same as step a) and b) of the claimed method for the prognosis of the outcome of an individual afflicted by an AML with no chromosomal abnormalities and treated by at least one anti-AML drug. Accordingly, what is abovementioned for these steps apply mutatis mutandis to the present method.

Surprisingly, the inventors discovered that the set of 23 genes used for prognosing the outcome of an individual afflicted by an AML with no chromosomal abnormalities and treated by at least one anti-AML drug, are also relevant to determine the responsiveness of the said individual to a DNA repair pathway inhibitor.

As a consequence, each DNA repair pathway group of genes as above defined are relevant to determine if the individual is likely to respond to an inhibitor of the DNA repair pathway they belong to. That is,

- the BER pathway group of genes is relevant to determine if the individual will respond to a BER pathway inhibitor,
- the FANC pathway group of genes is relevant to determine if the individual will respond to a FANC pathway inhibitor,
- the HRR pathway group of genes is relevant to determine if the individual will respond to a HRR pathway inhibitor,
- the MMR pathway group of genes is relevant to determine if the individual will respond to a MMR pathway inhibitor and
- the NER pathway group of genes is relevant to determine if the individual will respond to a NER pathway inhibitor.

In one embodiment, if at least one $Score_{PATHJ}$ **is** higher than the respective reference value, then the method comprises a step d) of administering the respective at least one DNA repair pathway inhibitor.

In one embodiment, if the individual is likely to respond to a BER pathway inhibitor, then the method comprises a step d) of administrating a BER pathway inhibitor to the individual. The said BER pathway inhibitor can be chosen from the group comprising PARP inhibitor, Ape1 DNA repair & redox inhibitor, Pol β inhibitor, Wee1 inhibitor and a combination thereof. The PARP inhibitor can be chosen from the group comprising INO-1001, AG14361, AG014699, ABT-888, AZD2281 and a combination thereof. The Ape1 DNA repair & redox inhibitor can be chosen from the group comprising Methoxyamine, 7-nitroindole-2-carboxylic acid, Lucanthone, arylstibonic acid compound 13755, E3330 and a combination thereof. The Pol β inhibitor can be chosen from the group comprising KA-A, Stigmasterol, Oleanolic acid, edgeworin, betulinic acid and a combination thereof. The Wee1 inhibitor can be chosen from 681640 and AZD1775. The BER pathway inhibitor may also be chosen in the group comprising TRC102, CEP-8933, NSC-281680, pamoic acid, eicosapentaenoic acid, L67 and L189, CRT0044876, AG014688 (also known as CO-338 and rucaparib), MK4827, CEP-9722, GPI-21016 (also known as E7016), BMN673, and BSI-201 (also known as iniparib).

In one embodiment, if the individual is likely to respond to a FANC pathway inhibitor, then the method comprises a step d) of administrating a FANC pathway inhibitor to the individual. The said FANC pathway inhibitor can be an inhibitor of FANCD2 monoubiquitylation such as proteasome inhibitors bortezomib and MG132, curcumin, and the curcumin analogues EF24 and 4H-TTD. The said FANC pathway inhibitor may be chosen from the compound described in WO2008066624, US7858331.

In one embodiment, if the individual is likely to respond to a HRR pathway inhibitor, then the method comprises a step d) of administrating a HRR pathway inhibitor to the individual. The said HRR pathway inhibitor can be chosen from the group comprising MCI13E, B02, RI-1, Mirin, B02, A03, A10 imatinib, AG024322, and CDK1 inhibitors such as SCH727965.

In one embodiment, if the individual is likely to respond to a MMR pathway inhibitor, then the method comprises a step d) of administrating a MMR pathway inhibitor to the individual. The said MMR pathway inhibitor can be chosen from the group comprising Lomeguatrib, O6-benzylguanine, DAC and Wee1 inhibitor. The Wee1 inhibitor can be chosen from 681640 and AZD1775.

In one embodiment, if the individual is likely to respond to a NER pathway inhibitor, then the method comprises a step d) of administrating a NER pathway inhibitor to the individual. The said NER pathway inhibitor can be chosen from the group comprising F11782, Cyclosporine, Cetuximab and Weee1 inhibitor. The Wee1 inhibitor can be chosen from 681640 and AZD1775.

A method for treating an individual afflicted by an acute myeloid leukaemia with no chromosomal abnormalities, treated by at least one drug used for treating acute myeloid leukaemia and able to respond to a DNA repair pathway inhibitor is also disclosed which is not part of the present invention, said method comprising the following steps:

a) identifying the patient able to respond to a DNA pathway inhibitor by the following sub steps:

i) measuring in a biological sample from said individual, the expression level of the 23 genes consisting of the nucleic acid sequences SEQ ID NO:1 to SEQ ID NO:23;
ii) calculating J scores according to the following formula

$$Score_{PATHJ} = \sum_{i=k1}^{kn} \beta i \times Ci$$

wherein $\beta i$ represents the regression $\beta$ coefficient reference value for the gene of nucleic acid sequence SEQ ID NO:i,

wherein $Ci = 1$ if the expression level of the gene of nucleic acid sequence SEQ ID NO:i is higher than an expression level of reference $ELR_i$ or $Ci = -1$ if the expression level of the gene of nucleic acid sequence SEQ ID NO:i is lower than or equal to $ELR_i$,

wherein J is an integer from 1 to 5 defining five scores $Score_{PATH1}$ to $Score_{PATH5}$,

wherein $Score_{PATH1}$ is calculated according to the expression level of genes of nucleic acid sequences SEQ ID NO:A1 to SEQ ID NO:A4 and wherein k is A and n is 4,

$Score_{PATH2}$ is calculated according to the expression level of genes of nucleic acid sequences SEQ ID NO:B1 to SEQ ID NO:B6 and wherein k is B and n is 6,

$Score_{PATH3}$ is calculated according to the expression level of genes of nucleic acid sequences SEQ ID NO:C1 to SEQ ID NO:C6 and wherein k is C and n is 6,

$Score_{PATH4}$ is calculated according to the expression level of genes of nucleic acid sequences SEQ ID NO:D1 to SEQ ID NO:D3 and wherein k is D and n is 3, and

$Score_{PATH5}$ is calculated according to the expression level of genes of nucleic acid sequences SEQ ID NO:E1 to SEQ ID NO:E8 and k is E and n is 8;

iii) confirming that the at least one calculated $Score_{PATHJ}$ is higher than a reference value $Ref_J$
iv) concluding that the individual is able to respond to the DNA repair pathway inhibitor,

b) administering the DNA repair pathway inhibitor to the individual.

In one embodiment, step a) is identifying the patient able to respond to a BER pathway inhibitor, wherein $Score_{PATH1}$ is calculated, wherein it is confirmed that $Score_{PATH1}$ is higher than $Ref_1$, wherein it is concluded that the individual is able to respond to a BER pathway inhibitor and step b) is administering a BER pathway inhibitor to the individual.

In one embodiment, step a) is identifying the patient able to respond to a FANC pathway inhibitor, wherein $Score_{PATH2}$ is calculated, wherein it is confirmed that $Score_{PATH2}$ is higher than $Ref_2$, wherein it is concluded that the individual is able to respond to a FANC pathway inhibitor and step b) is administering a FANC pathway inhibitor to the individual.

In one embodiment, step a) is identifying the patient able to respond to a HRR pathway inhibitor, wherein $Score_{PATH3}$ is calculated, wherein it is confirmed that $Score_{PATH3}$ is higher than $Ref_3$, wherein it is concluded that the individual is able to respond to a HRR pathway inhibitor and step b) is administering a HRR pathway inhibitor to the individual.

In one embodiment, step a) is identifying the patient able to respond to a MMR pathway inhibitor, wherein $Score_{PATH4}$ is calculated, wherein it is confirmed that $Score_{PATH4}$ is higher than $Ref_4$, wherein it is concluded that the individual is able to respond to a MMR pathway inhibitor and step b) is administering a MMR pathway inhibitor to the individual.

In one embodiment, step a) is identifying the patient able to respond to a NER pathway inhibitor, wherein $Score_{PATH5}$ is calculated, wherein it is confirmed that $Score_{PATH5}$ is higher than $Ref_5$, wherein it is concluded that the individual is able to respond to a NER pathway inhibitor and step b) is administering a NER pathway inhibitor to the individual.

A composition comprising at least one drug used for treating acute myeloid leukaemia and at least one DNA repair pathway inhibitor, is also disclosed which is not part of the present invention. Advantageously, the at least one DNA repair pathway inhibitor is selected in the group comprising BER pathway inhibitors, FANC pathway inhibitors, HRR pathway inhibitors, MMR pathway inhibitors, NER pathway inhibitors and a combination thereof.

A composition comprising at least one DNA repair pathway inhibitor for its use for the treatment of an individual afflicted by an acute myeloid leukaemia with no chromosomal abnormalities having a bad outcome as identified by the method abovementioned, belonging to the median risk group or the high risk group as identified by the method abovementioned or belonging to the group C, to the group B or to the group A and the median risk group as identified by the method abovementioned, is also disclosed which is not part of the present invention.

In one embodiment, the composition comprises a Base Excision Repair pathway inhibitor and the individual is likely to respond to a Base Excision Repair pathway inhibitor as identified by the method according to the method above mentioned.

In one embodiment, the composition comprises a Fanconi pathway inhibitor and the individual is likely to respond to a Fanconi pathway inhibitor as identified by the method above mentioned.

In one embodiment, the composition comprises a Homologous Recombination Repair pathway inhibitor and the individual is likely to respond to a Homologous Recombination Repair pathway inhibitor as identified by the method above mentioned.

In one embodiment, the composition comprises a Mismatch Repair pathway inhibitor and the individual is likely to respond to a Mismatch Repair pathway inhibitor as identified by the method above mentioned.

In one embodiment, the composition comprises a Nucleotide Excision Repair pathway inhibitor and the individual is likely to respond to a Nucleotide Excision Repair pathway inhibitor as identified by the method above mentioned.

In one embodiment, the composition comprises a Homologous Recombination Repair pathway inhibitor and a Nucleotide Excision Repair pathway inhibitor, wherein the individual belongs to the high-risk group, as identified by the method according to claim 2, and wherein the individual is likely to respond to a Homologous Recombination Repair pathway inhibitor and a Nucleotide Excision Repair pathway inhibitor as identified by the method above mentioned.

The composition above mentioned may comprise the 30 following formulations:

| Formulation | BER pathway inhibitor | FANC pathway inhibitor | MMR pathway inhibitor | HRR pathway inhibitor | NER pathway inhibitor |
|---|---|---|---|---|---|
| 1 | + | | | | |
| 2 | | + | | | |
| 3 | | | + | | |
| 4 | | | | + | |
| 5 | | | | | + |
| 6 | + | + | | | |
| 7 | + | | + | | |
| 8 | + | | | + | |
| 9 | + | | | | + |
| 10 | | + | + | | |
| 11 | | + | | + | |
| 12 | | + | | | + |
| 13 | | | + | + | |
| 14 | | | + | | + |
| 15 | | | | + | + |
| 16 | + | + | + | | |
| 17 | + | + | | + | |
| 18 | + | + | | | + |
| 19 | + | | + | + | |
| 20 | + | | + | | + |
| 21 | + | | | + | + |
| 22 | | + | + | + | |
| 23 | | + | + | | + |
| 24 | | + | | + | + |
| 25 | | | + | + | + |

(continued)

| Formulation | BER pathway inhibitor | FANC pathway inhibitor | MMR pathway inhibitor | HRR pathway inhibitor | NER pathway inhibitor |
|---|---|---|---|---|---|
| 26 | + | + | + | + | |
| 27 | + | + | + | | + |
| 28 | + | + | | + | + |
| 29 | | + | + | + | + |
| 30 | + | + | + | + | + |

In an advantageous embodiment, the invention relates to the composition as defined above, in association with at least one drug used for treating acute myeloid leukaemia, and possibly for which some resistance occurs.

In the invention, "a drug commonly used for treating acute myeloid leukaemia" refers to anticancer drugs or compounds.

Advantageously, the at least one drug used for treating acute myeloid leukaemia chosen from daunorubicin, idarubicin, cytarabine, midostaurin, cladribine, gemtuzumab ozogamicin, and a combination thereof.

Advantageously, the composition as defined above comprises said drug for treating AML and said inhibitor to be used simultaneously, separately, or sequentially.

By a simultaneous use, it is meant that all the compounds are injected or administered to an individual at the same time. Separately use means that the compounds are provided in a separate formulation but are injected or administered at the same time. Sequentially means that the compounds are delivered to the individual separately over the time.

The above inhibitors are able to induce apoptosis or to inhibit cell cycle of primary acute myeloid leukaemia cells from patients or acute myeloid leukaemia cell lines.

Resistance to a drug, regarding AML, means that said drug is not able to affect survival and/or proliferation of the cells that constitute AML. If a resistance occurs, it means that the AML was initially sensitive to the drug, but further to the treatment, or during the treatment, mutations may occur, and the target of the drugs are not any more sensitive to the drug. Therefore, the cells become insensitive to the drug and a resistance appears.

Finally, A composition comprising a drug used for treating acute myeloid leukemia, and possibly for which some resistance occurs, and at least one DNA repair pathway inhibitor, or a combination thereof, possibly in association with a pharmaceutically acceptable vehicle, is also disclosed whohc is not part of the present invention.

The composition comprises, in a pharmaceutical acceptable vehicle, an at least one inhibitor of at least one of the above listed DNA repair pathways and at least one of the above listed drugs for treating acute myeloid leukemia, in particular drugs for which resistance may occur.

It is within the skills of a physician to determine the specific therapeutically effective dosage regimen, as this dosage regimen will be dependent upon a variety of factors including, but not limited to: the severity of the acute myeloid leukemia; the age; the body weight; general health; the sex; the diet; the time course of administration; the route of administration; the duration of the treatment; the drugs that are concomitantly administered in combination with the pharmaceutical composition within the scope of the present invention.

In some embodiments, the dosage regimen said at least one inhibitor and said drug may range from about 0.0001 mg to about 1,000 mg per adult, per day. Preferably, the individual is administered with an amount of about 0.0001, 0.0005, 0.001, 0.005, 0.01, 0.05, 0.1, 0.5, 1.0, 2.5, 5.0, 7.5, 10.0, 15.0, 20.0, 25.0, 50.0, 75.0, 15 100, 250, 500 and 750 mg of said drug and said inhibitor in order to adjust the dosage regimen that is the most suitable to a particular individual in need of the treatment.

A pharmaceutical composition as defined abovemay contain from about 0.01 mg to about 500 mg of said drug and said at least one inhibitor, preferably from about 1 mg to about 100 mg of said drug and said at least one inhibitor.

In a preferred embodiment, an effective amount of said inhibitor and said at least one inhibitor is routinely administered to an individual in need thereof, at a dosage regimen from about 0.0002 mg/kg to about 20 mg/kg of body weight per day, in particular from about 0.001 mg/kg to 7 mg/kg of body weight per day.

The optimal amount of said inhibitor and said at least one inhibitor to be comprised in a pharmaceutical dosage unit according to the invention may be easily adapted by the one skilled in the art using routine known protocols or methods.

Said inhibitor and said at least one inhibitor and the pharmaceutical composition comprising thereof disclosed herein may be administered by any suitable route, i.e. including, but not limited to, an oral, sublingual, buccal, subcutaneous, transdermal, topical, intraperitoneal, intramuscular, intravenous, subdermal, intrathecal and intranasal and rectal administration.

LEGENDS TO THE FIGURES.

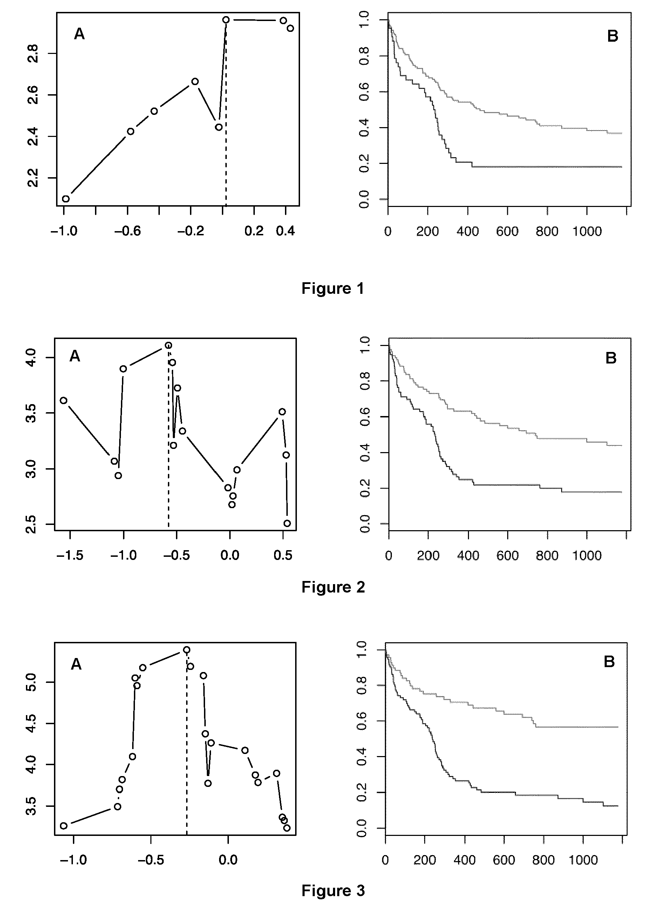

**Figure 1A** represents the determination of BER score cut-point. Patients of the training cohort (n=162) were ranked according to increasing BER scores (x-axis) and a maximum difference in OS was obtained using MaxStat R function. The cut-point value (trashed line) is 0.022276. **Figure 1B** is a Kaplan Meier curve showing the percentage of survival of the patients vs time (days). Top survival curve (1) is for patients (n=120) whose score is inferior or equal to the MaxStat determined cut-point. Bottom survival curve (2) is for patients (n=42) whose score is strictly superior to the MaxStat determined cut-point. p-value=0.00117.

**Figure 2A** represents the determination of FANC score cut-point. Patients of the training cohort (n=162) were ranked according to increasing FANC scores (x-axis) and a maximum difference in OS was obtained using MaxStat R function. The cut-point value (trashed line) is -0.57621. **Figure 2B** is a Kaplan Meier curve showing the percentage of survival of the patients vs time (days). Top survival curve is for patients (n=86) whose score is inferior or equal to the MaxStat determined cut-point. Bottom survival curve is for patients (n=76) whose score is strictly superior to the MaxStat determined cut-point. p-value=$1.79e^{-5}$.

**Figure 3A** represents the determination of HER score cut-point. Patients of the training cohort (n=162) were ranked according to increasing HER scores (x-axis) and a maximum difference in OS was obtained using MaxStat R function. The cut-point value (trashed line) is -0.26745. **Figure 3B** is a Kaplan Meier curve showing the percentage of survival of the patients vs time (days). Top survival curve is for patients (n=69) whose score is inferior or equal to the MaxStat determined cut-point. Bottom survival curve is for patients (n=93) whose score is strictly superior to the MaxStat determined cut-point. p-value=$5.08e^{-8}$.

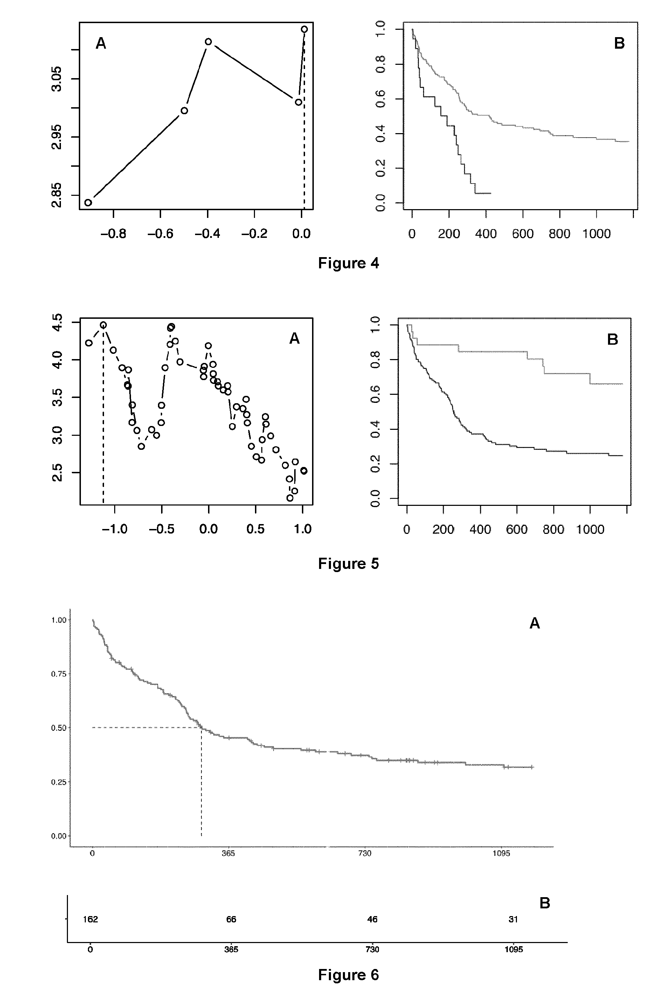

**Figure 4A** represents the determination of MMR score cut-point. Patients of the training cohort (n=162) were ranked according to increasing MMR scores (x-axis) and a maximum difference in OS was obtained using MaxStat R function. The cut-point value (trashed line) is -0.012234. **Figure 4B** is a Kaplan Meier curve showing the percentage of survival of the patients vs time (days). Top survival curve is for patients (n=144) whose score is inferior or equal to the MaxStat determined cut-point. Bottom survival curve is for patients (n=18) whose score is strictly superior to the MaxStat determined cut-point. p-value=$8.01e^{-5}$.

**Figure 5A** represents the determination of NER score cut-point. Patients of the training cohort (n=162) were ranked according to increasing NER scores (x-axis) and a maximum difference in OS was obtained using MaxStat R function. The cut-point value (trashed line) is -1.1213. **Figure 5B** is a Kaplan Meier curve showing the percentage of survival of the patients vs time (days). Top survival curve is for patients (n=26) whose score is inferior or equal to the MaxStat determined cut-point. Bottom survival curve is for patients (n=136) whose score is strictly superior to the MaxStat determined cut-point. p-value=$9.95e^{-5}$.

**Figure 6A** is a Kaplan Meier curve showing the percentage of survival of all the patients of the training cohort vs time (days). Trashed line represents the median overall survival. **Figure 6B** represents the number of patients at risk of the training cohort accounted at time intervals of one year (in days). Subjects who have died, dropped out, or move out are not counted as "at risk" i.e., subjects who are lost are not counted. Therefore, the patients at risk represent the remaining patients in the cohort at each time interval and not the overall surviving patients of the cohort.

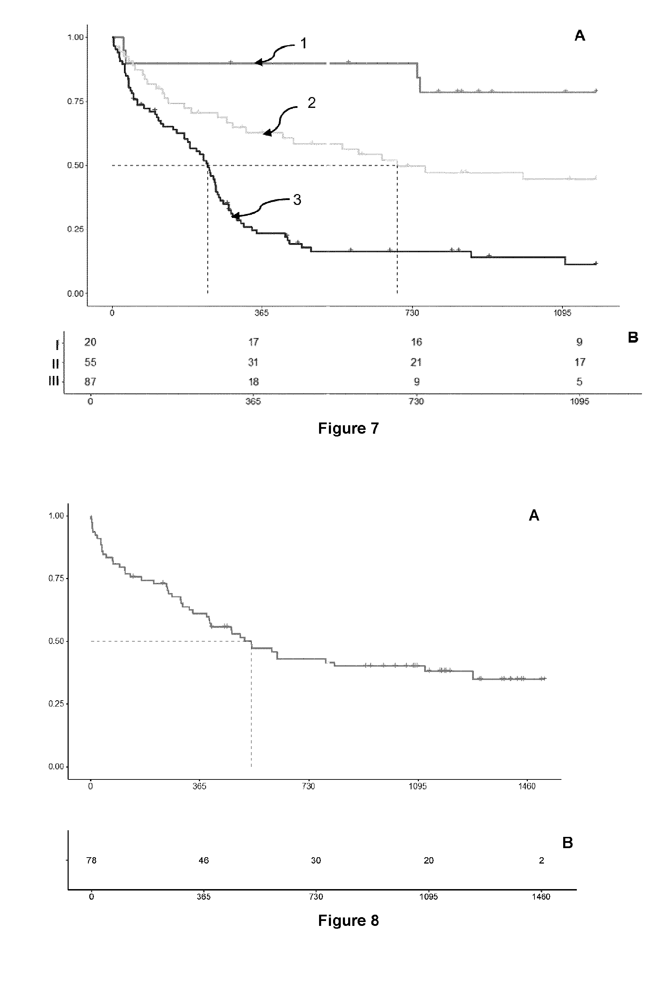

**Figure 7A** is a Kaplan Meier curve showing the percentage of survival of the patients of the training cohort vs time (days). Top survival curve (1) is for patient of Group I. Middle survival curve (2) is for patients of Group II. Bottom survival curve (3) is for patients of Group III. P-values are estimated with log-rank test: p-value between top and middle curves is equal to 0.016; p-value between middle and bottom curves is inferior to 0.001. Trashed line represents the median overall survival. **Figure 7B** represents the number of patients at risk of each Group I-III accounted at time intervals of one year (in days).

**Figure 8A** is a Kaplan Meier curve showing the percentage of survival of all the patients of the validation cohort vs time (days). Trashed line represents the median overall survival (293 days). **Figure 8B** represents the number of patients at risk of the validation cohort accounted at time intervals of one year (in days).

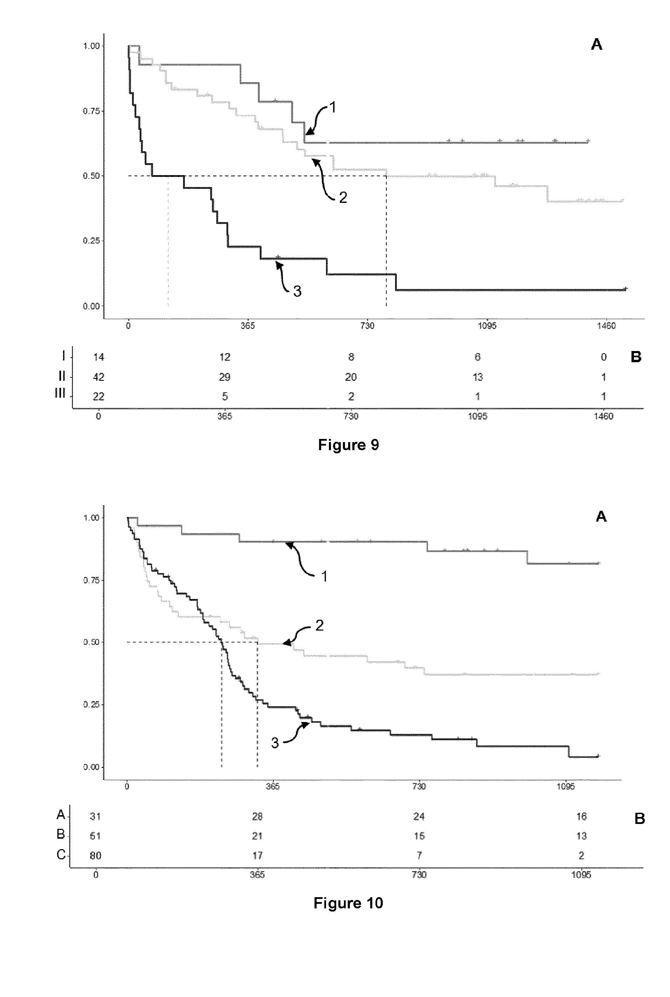

**Figure 9A** is a Kaplan Meier curve showing the percentage of survival of the patients of the validation cohort vs time (days). Top survival curve (1) is for patient of Group I. Middle survival curve (2) is for patients of Group II. Bottom survival curve (3) is for patients of Group III. P-values are estimated with log-rank test: p-value between top and middle curves is equal to 0.287; p-value between middle and bottom curves is inferior to 0.001. Trashed line represents the median overall survival. **Figure 9B** represents the number of patients at risk of each Group I-III accounted at time intervals of one year (in days).

**Figure 10A** is a Kaplan Meier curve showing the percentage of survival of the patients of the training cohort vs time (days). Top survival curve (1) is for patient of Group A. Middle survival curve (2) is for patients of Group B. Bottom survival curve (3) is for patients of Group C. P-values are estimated with log-rank test: p-value between top and middle curves is inferior to 0.001; p-value between middle and bottom curves is equal to 0.004. Trashed line represents the median overall survival. **Figure 10B** represents the number of patients at risk of each Group A-C accounted at time intervals of one year (in days).

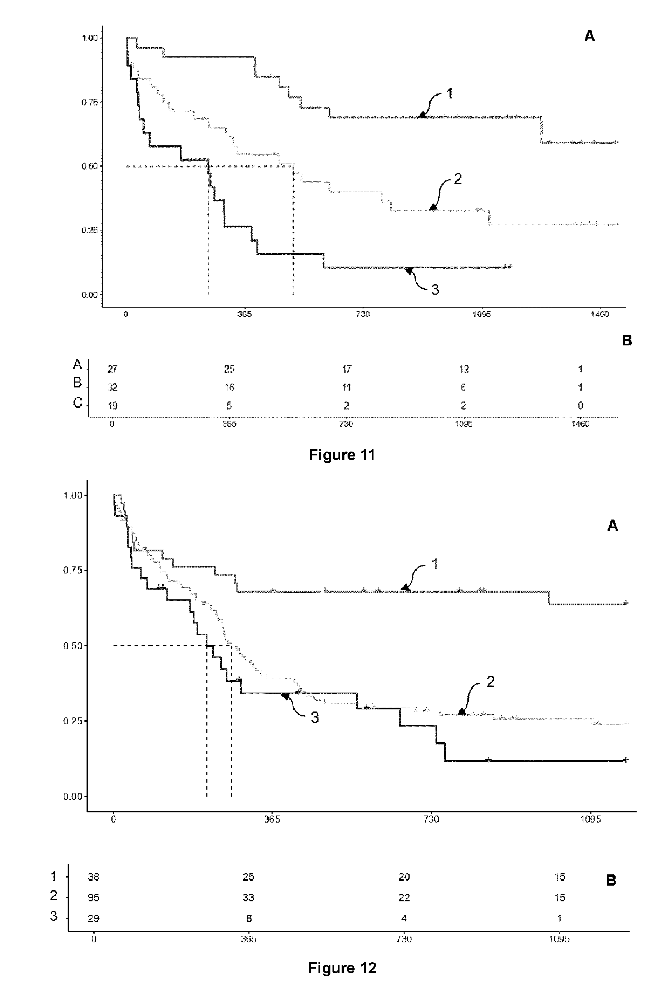

**Figure 11A** is a Kaplan Meier curve showing the percentage of survival of the patients of the validation cohort vs time (days). Top survival curve (1) is for patient of Group A. Middle survival curve (2) is for patients of Group B. Bottom survival curve (3) is for patients of Group C. P-values are estimated with log-rank test: p-value between top and middle curves is equal to 0.005; p-value between middle and bottom curves is equal to 0.023. Trashed line represents the median overall survival. **Figure 11B** represents the number of patients at risk of each Group A-C accounted at time intervals of one year (in days).

**Figure 12A** is a Kaplan Meier curve showing the percentage of survival of the patients of the training cohort vs time (days). Top survival curve (1) is for patient with NPM1+/FLT3-ITD- mutational status. Middle survival curve (2) is for patients with NPM1+/FLT3-ITD+ mutational status. Bottom survival curve (3) is for patients with NPM1-/ FLT3-ITD+ mutational status. P-values are estimated with log-rank test: p-value between top and middle curves is equal to 0.001; p-value between middle and bottom curves is equal to 0.241. Trashed line represents the median overall survival. **Figure 12B** represents the number of patients at risk for each mutational status (1, 2 and 3) accounted at time intervals of one year (in days).

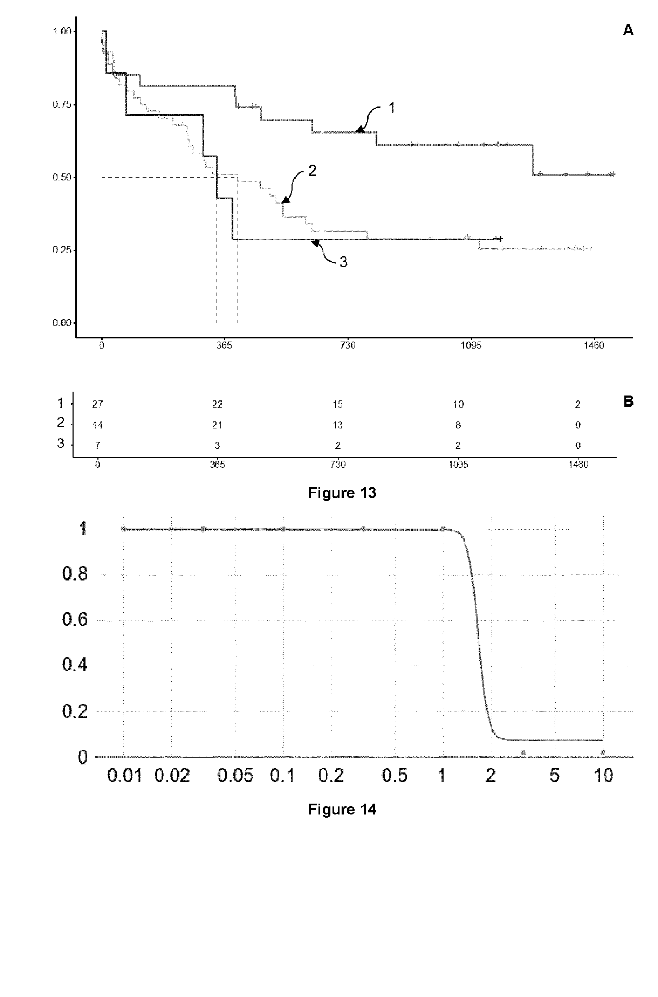

**Figure 13A** is a Kaplan Meier curve showing the percentage of survival of the patients of the validation cohort vs time (days). Top survival curve (1) is for patient with NPM1+/FLT3-ITD- mutational status. Middle survival curve (2) is for patients with NPM1+/FLT3-ITD+ mutational status. Bottom survival curve (3) is for patients with NPM1-/ FLT3-ITD+ mutational status. P-values are estimated with log-rank test: p-value between top and middle curves is equal to 0.016; p-value between middle and bottom curves is equal to 0.946. Trashed line represents the median overall survival. **Figure 13B** represents the number of patients at risk with each mutational status (1, 2 and 3) accounted at time intervals of one year (in days).

Figure 14 represents a graph evidencing the effect of Wee1 inhibitor concentration (in micromolar; x-axis) on viability of AML cell line OCIAML2 (y-axis).

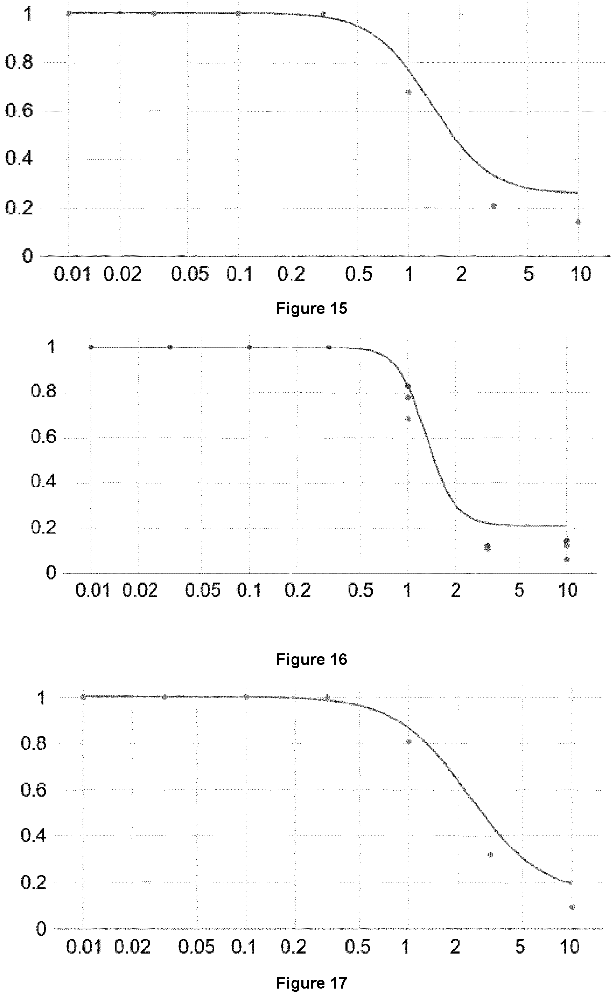

Figure 15 represents a graph evidencing the effect of Wee1 inhibitor concentration (in micromolar; x-axis) on viability of AML cell line OCIAML3 (y-axis).

Figure 16 represents a graph evidencing the effect of Wee1 inhibitor concentration (in micromolar; x-axis) on viability of AML cell line OCIM1 (y-axis).

Figure 17 represents a graph evidencing the effect of Wee1 inhibitor concentration (in micromolar; x-axis) on viability of AML cell line HL60 (y-axis).

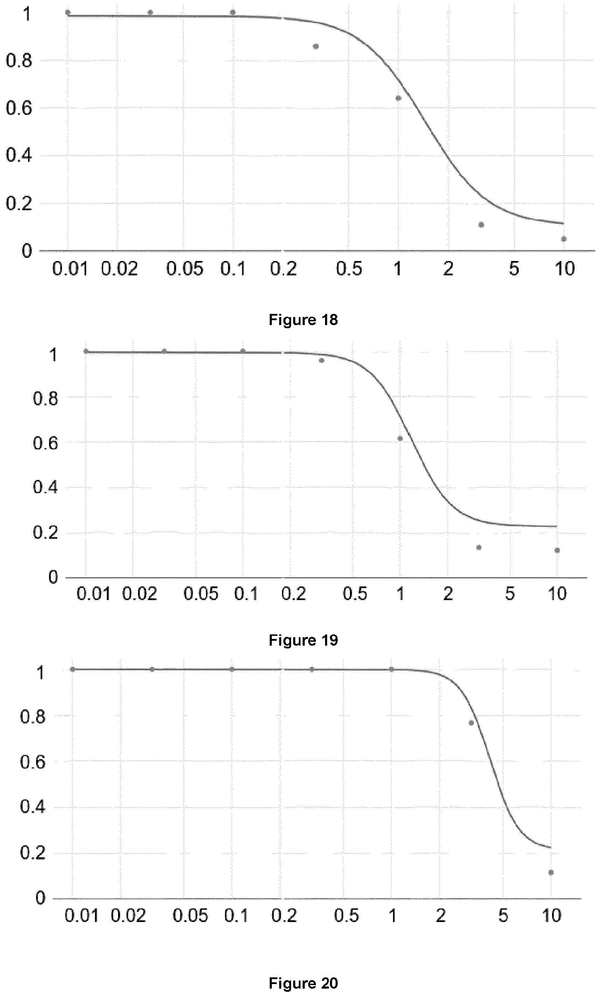

Figure 18 represents a graph evidencing the effect of Wee1 inhibitor concentration (in micromolar; x-axis) on viability of AML cell line KY821 (y-axis).

Figure 19 represents a graph evidencing the effect of Wee1 inhibitor concentration (in micromolar; x-axis) on viability of AML cell line GDM1 (y-axis).

Figure 20 represents a graph evidencing the effect of Wee1 inhibitor concentration (in micromolar; x-axis) on viability of AML cell line KMOE2 (y-axis).

**EXEMPLE**

## 1. Patients and Method

### Patients and gene expression data

Gene expression microarray data from two independent cohorts of adult patients diagnosed with CN-AML were used. The first cohort (training set) included 162 patients and the second one (validation set) 78 patients. At least 20 metaphases were analyzed for each patient to confirm the normal karyotype. At the beginning of treatment, median age was 58 years in the training cohort and 62 years in the validation cohort. Pretreatment clinical characteristics of patients have been described previously (Metzeler KH et al., Blood 2008;112:4193-201). *NPM1* and *FLT3* mutational status were kindly provided for each patient by Metzeler *et al.* All patients were treated with intensive chemotherapy which consists in an induction chemotherapy regimen using high-dose cytarabine (excepted for one patient which was treated with anthracycline), followed by consolidation chemotherapy and either autologous stem cell transplantation or prolonged monthly maintenance chemotherapy (details are available at https://clinicaltrials.gov/ct2/show/study/NCT00266136).

Affymetrix gene expression data are publicly available via the online Gene Expression Omnibus (http://www.ncbi.nlm.nih.gov/geo/) under accession number GSE12417. They were performed using Affymetrix HG-U133 A&B microarrays for first (training) cohort and Affymetrix HG-U133 plus 2.0 microarrays for the second (validation) one. Normalization of microarray data was performed using the variance stabilizing normalization algorithm, and probe set signals calculated by the median polish method. Quality control consisted of visual inspection of the array image for artifacts, assessment of RNA degradation plots, and inspection of rank-vs-residual plots after normalization and probe set summarization.

### Selection of prognostic genes

A list of 175 genes involved in the six major DNA repair pathways base excision repair (BER), nucleotide excision repair (NER), mismatch repair (MMR), homologous recombination repair (HRR), non-homologous end joining (NHEJ) and FANC pathways] was defined using the REPAIRtoire database (http://repairtoire.genesilico.pl) and review of the literature. To establish gene expression (GE)-based risk scores, the inventors selected probe sets whose expression values were significantly associated with overall survival, using MaxStat R function and Benjamini Hochberg multiple testing correction (adjusted p-value < 0.05).

### Building gene expression-based risk score on training cohort

For each pathway, a GE-based risk score was created as the sum of the beta coefficients weighted by +1 or -1 according to the patient signal above or below / equal the probe set MaxStat value. Patients from the training cohort were ranked according to increased prognostic score and for a given score value X, the difference in survival of patients with a prognostic score ≤X or >X was computed using MaxStat analysis.

Cox proportional hazards model was performed to determine statistically significant pathway scores in multivariate analysis. A global DNA repair score was calculated based on the pathway scores which remained statistically significant in this analysis. Survival analyses were assessed using Kaplan-Meier method, and survival curves were compared using log-rank test.

### Validation of the DNA repair score on validation cohort

Pathway and DNA repair scores were individually calculated in the validation cohort, using the cutoff values determined for the training cohort. Survival analyses were assessed using Kaplan-Meier method, and survival curves were compared using log-rank test.

### Statistical analyses

All statistical tests were two-tails and Alpha-risk was fixed at 5%. Analyses were performed using R.3.6.0. and SPSS Statistics version 23.0.0.0 for Mac.

## 2. Results

### Probe sets selection

Considering the important role of DNA repair in drug resistance and adaptation to replication stress in cancer cells, the inventors first aimed to identify the DNA repair genes associated with overall survival in CN-AML. Using the MaxStat R function, the inventors identified 23 out of the 175 genes with a prognostic value in the two independent cohorts. Nineteen genes were associated with poor prognosis and 4 genes with good prognosis. No statistically significant prognostic value was found for any gene involved in NHEJ pathway.

Risk scores and survival analysis in training cohort

Then, the inventors combined the prognosis information of these genes in a GE-based DNA repair risk score. A specific GE-based risk score was established for BER, FANC, HRR, MMR and NER DNA repair pathways. GE-based DNA repair scores were defined by the sum of the beta coefficients of the Cox model for each prognostic gene, weighted by +1 or -1 according to the patient signal above or below / equal the probe set MaxStat value. High BER, FANC, HRR, MMR and NER scores were significantly associated with poor prognosis in the training cohort (Figures 1 to 5). Accordingly, all the scores are associated with overall survival in the training cohort.

In Cox multivariate analysis, only HRR and NER scores remained associated with overall survival in the training cohort (Table 2). Cox multivariate analysis tests the independence of each score, i.e. if a score is associated to different patients from the other scores. The results presented in Table 2 shows that only HRR and NER scores are associated to different high-risk patients from the other scores, and thus remain associated with overall survival. Consequently, combining these two scores is relevant to improve the classification of the patients, as described below.

**Table 2**

| DNA repair pathway score | HR | p-value |
|---|---|---|
| BER score | 0.932 [0.561-1.548] | NS |
| FANC score | 1.298 [0.793-2.123] | NS |
| HRR score | 2.357 [1.445-3.845] | 0.001 |
| MMR score | 1.578 [0.864-2.883] | NS |
| NER score | 2.542 [1.185-5.453] | 0.017 |

*Legend to the Table: P-values and hazard ratio (HR) are shown for each DNA repair pathway score. 95% confidence intervals are displayed for HR. NS: not significant at a 5% threshold.*

Therefore, a global DNA repair score was established, incorporating the prognostic value of HRR and NER scores. To this aim, CN-AML patients were split in three subgroups: group I included patients with low NER and HRR risk scores (n=20), group III included patients with high NER and HRR risk scores (n=87) and group II included patients with one NER or HRR high risk value (n=55).

After a median follow-up of 1176 days (95% of the confident interval (CI): 916 days-not reached (NR)), median overall survival (OS) was 293 days (95% CI: 252-461) for the whole training cohort (Figure 6). One-year OS was 45.2% (95% CI: 38.0-53.8). According to risk groups determined by our DNA repair score, median OS was not reached (95% CI: NR-NR), 693 days (95% CI: 414-NR) and 233 days (95% CI: 184-260) respectively for patients in groups I, II and III (Figure 7). Median OS were statistically different between each risk group (log-rank test; p = 0.016 between group I and II; p < 0.001 between group II and III).

Risk scores and survival analysis in validation cohort

In the validation cohort, high and low values for HRR and NER scores were calculated for each patient with the cutoff values the inventors determined for the training cohort. The global DNA repair score was individually calculated. In this validation set, risk groups included 14, 42 and 22 patients respectively in groups I, II and III.

After a median follow-up of 1183 days (95% CI: 1092-1383), median overall survival (OS) was 538 days (95% CI: 388-1278) for the whole validation cohort (Figure 8). One-year OS was 61.1% (95% CI: 51.1-73.0). According to risk groups determined by the DNA repair score, median OS was not reached (95% CI: 538-NR), 787 days (95% CI: 473-NR) and 120 days (95% CI: 36-303) respectively for patients in groups I, II and III (Figure 9). Even if survival analysis failed to demonstrate a statistical difference between groups I and II (log-rank test; p = 0.287), OS was still statistically different between risk groups II and III (log-rank test; p < 0.001). Altogether, these data underlined the identification of high-risk CN-AML patients characterized by DNA repair dysregulation and that benefit from DNA repair targeted treatment.

DNA repair score and NPM1 / FLT3 mutational status combination as prognosis factors in CN-AML

Kaplan-Meier survival curves according to NPM1 and FLT3 mutational status are given in Figures 12A and 13A for both cohorts. In the training cohort (Figure 12), median OS was not reached (95% CI: 999-NR) for patients with NPM1+/FLT3-ITD- mutational status. Besides, median OS is 271 days (95% CI: 240-416) for patients with NPM1+/FLT3-ITD+ or NPM1-/FLT3- mutational status and 214 days (95% CI: 123-657) for patients with NPM1-/ FLT3-ITD+ mutational status. In the validation cohort (Figure 13), median OS was not reached (95% CI: 624-NR) for patients with NPM1+/FLT3-ITD-mutational status. Besides, median OS is 403 days (95% CI: 259-624) for patients with NPM1+/FLT3-ITD+ or NPM1-/FLT3- mutational status and 342 days (95% CI: 72-NR) for patients with NPM1-/ FLT3-ITD+ mutational status.

Because NPM1 mutations and FLT3-ITD are well-described prognosis factors in CN-AML, the inventors conducted another Cox multivariate analysis to determine whether our DNA repair score provides additional prognostic information compared to NPM1 mutations and FLT3-ITD. When compared for the training cohort, all of these three parameters were independently associated with survival.

| Parameter | HR | p-value |
|---|---|---|
| DNA repair score | 2.645 [1.840-3.801] | < 0.001 |
| *NPM1* mutation | 0.576 [0.385-0.862] | 0.007 |
| *FLT3*-ITD mutation | 1.773 [1.163-2.704] | 0.008 |

As expected, a high DNA repair score and *FLT3*-ITD were associated with a bad prognosis, whereas NPM1 mutation was associated with a favorable outcome.

Therefore, the inventors investigated the interest of combining DNA repair score and NPM1 / FLT3 mutational status to predict CN-AML outcome. Patients were classified according to prognosis value of DNA repair score (0 point for group I; 1 for group II; 2 for group III), and NPM1 / FLT3 mutational status (0 point if NPM1 mutated without *FLT3*-ITD; 2 points if *FLT3*-ITD without NPM1 mutation; 1 point in other situations). The sum of the prognostic information was computed for all patients, allowing to separate patients in three new prognosis groups: group A for patients with 0 or 1 point, group B for patients with 2 points and group C for patients with 3 or 4 points.

| | | Classification according to DNA repair score | | |
|---|---|---|---|---|
| | | Group I *(0 point)* | Group II *(1 point)* | Group III *(2 points)* |
| **NPM1 and FLT3 mutational status** | *NPM1+* and *FLT3*-ITD-<br>*(0 point)* | **Group A**<br>*(0 point)* | **Group A**<br>*(1 point)* | **Group B**<br>*(2 points)* |
| | *NPM1+* and *FLT3*-ITD+ *or NPM1-* and *FLT3*-ITD-*(1 point)* | **Group A** *(1 point)* | **Group B** *(2 points)* | **Group C** *(3 points)* |
| | *NPM1-* and *FLT3*-ITD+ *(2 points)* | **Group B** *(2 points)* | **Group C** *(3 points)* | **Group C** *(4 points)* |

In the training cohort, median OS was not reached (95% CI: NR-NR), 326 days (95% CI: 127-NR) and 236 days (95% CI: 190-263) respectively for patients in groups A, B and C. One-year OS was 90.3% (95% CI: 80.5-100) in group A, 49.3% (95% CI: 37.1-65.7) in group B, and 24.2% (95% CI: 16.2-36.2) in group C. These results were confirmed in the validation cohort: median OS was not reached (95% CI: 1278-NR), 516 days (95% CI: 308-NR) and 253 days (95% CI: 52-403) for patients respectively in groups A, B and C, with a one-year OS of 92.6% (95% CI: 83.2-100) in group A, 54.9% (95% CI: 39.8-75.7) in group B, and 26.5% (95% CI: 12.4-55.8) in group C. OS was statistically different between groups A, B and C in both training and validation cohorts (Figures 10-11).

## 3. AML cell lines dependency to DNA repair pathways

Hereafter is reported AML cell lines dependency to DNA repair pathways from data collected by The Genomics of Drug Sensitivity in Cancer Project repository.

AML cell lines (CTV-1, HL-60, GDM-1, HEL, KG-1, KMOE-2, KY821, ML-2, MONO-MAC-6, NKM-1, NOMO-1, P31-FUJ, THP-1, QIMR-WIL, CMK, CESS, OCI-AML2, OCI-AML3, NB4, ME-1, MOLM-13, MOLM-16, OCI-AML5, OCI-M1 and PL-21) were treated with two inhibitors, a PARP inhibitor (Olaparib) and a Wee1 inhibitor (681640). Cells were seeded in 384-well microplates at 15% confluency in culture medium with 10% FBS and Penicillin/Streptomycin. Cells were treated with compound immediately following plating, returned to the incubator for a 72-h time point, then stained with 55 $\mu$g/ml Resazurin (Sigma) prepared in Glutathione-free media for 4 hours. Quantitation of fluorescent signal intensity is performed using a fluorescent plate reader at excitation and emission wavelengths of 630/695 nM for Syto60, and 535/595 nM for Resazurin.

A significant depends was identified for PARP inhibitor (p=0.0001) and Wee1 inhibitor (p=8.5E-07), as reported by the below table :

| Drug | Dataset | T-Statistic | P-Value |
|------|---------|-------------|---------|
| **Olaparib** | Drug sensitivity AUC (Sanger GDSC1) | -3,794709473 | 0,000162116 |
| **Wee1 Inhibitor** | Drug sensitivity AUC (Sanger GDSC2) | -4,965743548 | 8,55E-07 |

The dose response curve to the WEE1 inhibitor (681640) is shown for several of the abovementioned AML cell lines on Figures 14-20.

**Claims**

1. A method for the *in vitro* prognosis of the outcome of an individual afflicted by an acute myeloid leukaemia (AML) with no chromosomal abnormalities and treated by at least one anti-AML drug, said method comprising the following steps:

   a) measuring, in a biological sample from said individual, the expression level of all the 23 genes consisting of the nucleic acid sequences as set forth in SEQ ID NO:1 to SEQ ID NO:23;
   b) calculating J scores according to the following formula

$$Score_{PATHJ} = \sum_{i=k1}^{kn} \beta i \times Ci$$

   wherein $\beta i$ represents the regression $\beta$ coefficient reference value for the gene of nucleic acid sequence SEQ ID NO:i,
   wherein $Ci = 1$ if the expression level of the gene of nucleic acid sequence SEQ ID NO:i is higher than an expression level of reference $ELR_i$ or $Ci = -1$ if the expression level of the gene of nucleic acid sequence SEQ ID NO:i is lower than or equal to $ELR_i$,
   wherein J is an integer from 1 to 5 defining five scores $Score_{PATH1}$ to $Score_{PATH5}$,
   wherein $Score_{PATH1}$ is calculated according to the expression level of genes of nucleic acid sequences SEQ ID NO:A1 to SEQ ID NO:A4 and wherein k is A and n is 4,
   $Score_{PATH2}$ is calculated according to the expression level of genes of nucleic acid sequences SEQ ID NO:B1 to SEQ ID NO:B6 and wherein k is B and n is 6,
   $Score_{PATH3}$ is calculated according to the expression level of genes of nucleic acid sequences SEQ ID NO:C1 to SEQ ID NO:C6 and wherein k is C and n is 6,
   $Score_{PATH4}$ is calculated according to the expression level of genes of nucleic acid sequences SEQ ID NO:D1 to SEQ ID NO:D3 and wherein k is D and n is 3, and
   $Score_{PATH5}$ is calculated according to the expression level of genes of nucleic acid sequences SEQ ID NO:E1 to SEQ ID NO:E8 and k is E and n is 8;

   c) prognosing that:

   i. if at least one of the scores $Score_{PATHJ}$ **is** higher than a respective reference value $Ref_J$, then the individual is likely to have a bad outcome with a one-year survival (OYS) below 40%,
   ii. if all the scores $Score_{PATH1}$ to $Score_{PATH5}$ are lower than or equal to their respective reference value $Ref_1$ to $Ref_5$, then the individual is likely to have a good outcome with an OYS higher than 50%,
   iii. if the score $Score_{PATH1}$ is higher than its respective reference value $Ref_1$, then the individual is likely to have a bad outcome with an OYS below 20%,
   iv. if the score $Score_{PATH1}$ is lower than or equal to its respective reference value $Ref_1$, then the individual is likely to have a good outcome with an OYS higher than 55%,
   v. if the score $Score_{PATH2}$ is higher than its respective reference value $Ref_2$, then the individual is likely to have a bad outcome with an OYS below 25%,

vi. if the score $Score_{PATH2}$ is lower than or equal to its respective reference value $Ref_2$, then the individual is likely to have a good outcome with an OYS higher than 60%,

vii. if the score $Score_{PATH3}$ is higher than its respective reference value $Ref_3$, then the individual is likely to have a bad outcome with an OYS below 30%,

viii. if the score $Score_{PATH3}$ is lower than or equal to its respective reference value $Ref_3$, then the individual is likely to have a good outcome with an OYS higher than 70%,

ix. if the score $Score_{PATH4}$ is higher than its respective reference value $Ref_4$, then the individual is likely to have a bad outcome with an OYS below 10%,

x. if the score $Score_{PATH4}$ is lower than or equal to its respective reference value $Ref_4$, then the individual is likely to have a good outcome with an OYS higher than 50%,

xi. if the score $Score_{PATH5}$ is higher than its respective reference value $Ref_5$, then the individual is likely to have a bad outcome with an OYS below 40%,

xii. if the score $Score_{PATH5}$ is lower than or equal to its respective reference value $Ref_5$, then the individual is likely to have a good outcome with an OYS higher than 80%,

wherein the reference value for $Score_{PATH1}$ is $Ref_1$ and equals to 0.022276,
the reference value for $Score_{PATH2}$ is $Ref_2$ and equals to -0.57621,
the reference value for $Score_{PATH3}$ is $Ref_3$ and equals to -0.26745
the reference value for $Score_{PATH4}$ is $Ref_4$ and equals to 0.012234, and
the reference value for $Score_{PATH5}$ is $Ref_5$ and equals to -1.1213.

**2.** The method according to claim 1, wherein step c) further comprises the following prognosis

xiii. if both scores $Score_{PATH3}$ and $Score_{PATH5}$ are lower than their respective reference value $Ref_3$ and $Ref_5$, then the individual belongs to a low risk group with an OYS higher than 80%,

xiv. if one of the scores $Score_{PATH3}$ and $Score_{PATH5}$ is higher than its respective reference value $Ref_3$ or $Ref_5$, and the other one is lower than its respective reference value $Ref_3$ or $Ref_5$, then the individual belongs to a median risk group with an OYS between 60 and 80%,

xv. if both $Score_{PATH3}$ and $Score_{PATH5}$ are higher than their respective reference value $Ref_3$ and $Ref_5$, then the individual belongs to a high-risk group with an OYS below 30%.

**3.** The method according to claim 2, wherein the method further comprises the following steps

d) determining the mutational status of both the *NPM1* gene and *FLT3* gene, said *NPM1* gene having a nucleic acid sequence as set forth in SEQ ID NO:24 and said *FLT3* gene having a nucleic acid sequence as set forth in SEQ ID NO:25,

wherein if the *NPM1* gene is mutated such that it codes for a NMP1 protein which is delocalized into the cytoplasm, the individual is classified as *NPM1+,*
otherwise the individual is classified as *NPM1-,*
if the *FLT3* gene is mutated such that it codes for a FLT3 protein having an internal tandem duplication in the juxta-transmembrane domain, the individual which is classified as *FLT3*-ITD+,
otherwise the individual is classified as *FLT3*-ITD-;

e) classifying the individual such that

i. the individual belongs to a group A with an OYS higher than 85% if

- the individual is *NPM1+* and *FLT3*-ITD- and belongs to the low risk group or the median risk group according to step c), or
- the individual is classified *NPM1+* and *FLT3*-ITD+ or *NPM1-* and *FLT3*-ITD-, and belongs to the low risk group according to step c)

ii. the individual belongs to a group B with OYS between 40 and 60%, if

- the individual is classified *NPM1+* and *FLT3*-ITD- and belongs to the high-risk group according to step c),
- the individual is classified *NPM1+* and *FLT3*-ITD+ or *NPM1-* and *FLT3*-ITD-, and belongs to the median risk group according to step c), or
- the individual is classified NPM1- and *FLT3*-ITD+ and belongs to the low risk group according to step c)

iii. the individual belongs to a group C with an OYS below 30% if

- the individual is classified *NPM1+* and *FLT3*-ITD+ or *NPM1*- and *FLT3*-ITD-, and belongs to the high-risk group according to step c), or
- the individual is classified *NPM1*- and *FLT3*-ITD+ and belongs to the median risk group or the high-risk group according to step c).

4. The method according to anyone of claims 1 to 3, wherein the biological sample is selected in the group comprising a cell culture, a cell line, a tissue biopsy such as a bone marrow aspirate, a biological fluid such as a blood, pleural effusion and a serum sample.

5. The method according to anyone of claims 1 to 4, wherein the at least one drug used for treating acute myeloid leukaemia is chosen from daunorubicin, idarubicin, cytarabine, midostaurin, cladribine, gemtuzumab ozogamicin, and a combination thereof.

**Patentansprüche**

1. Verfahren zur *In-vitro*-Prognose des Behandlungsergebnisses bei einer Person, die an einer akuten myeloischen Leukämie (AML) ohne Chromosomenanomalien leidet und mit mindestens einem Anti-AML-Medikament behandelt wird, wobei das Verfahren die folgenden Schritte umfasst:

a) Messen des Expressionsniveaus aller 23 Gene, die aus den in SEQ ID NO:1 bis SEQ ID NO:23 angegebenen Nukleinsäure Sequenzen bestehen, in einer biologischen Probe der Person;
b) Berechnen von J-Werten gemäß der folgenden Formel

$$Score_{PATHJ} = \sum_{i=k1}^{kn} \beta i \times Ci$$

wobei $\beta i$ den Referenzwert des Regressionskoeffizienten $\beta$ für das Gen der Nukleinsäuresequenz SEQ ID NO:i darstellt,
wobei $Ci = 1$ ist, wenn das Expressionsniveau des Gens der Nukleinsäuresequenz SEQ ID NO:i höher ist als ein Referenz-Expressionsniveau $ELR_i$ oder $Ci = -1$ ist, wenn das Expressionsniveau des Gens der Nukleinsäuresequenz SEQ ID NO:i niedriger oder gleich $ELR_i$ ist,
wobei J eine ganze Zahl von 1 bis 5 ist, die fünf $Score_{PATH1}$ bis $Score_{PATH5}$ definiert,
wobei $Score_{PATH1}$ gemäß dem Expressionsniveau von Genen der Nukleinsäuresequenzen SEQ ID NO:A1 bis SEQ ID NO:A4 berechnet wird und wobei k A und n 4 ist,
$Score_{PATH2}$ gemäß dem Expressionsniveau von Genen der Nukleinsäuresequenzen SEQ ID NO:B1 bis SEQ ID NO:B6 berechnet wird und wobei k B und n 6 ist,
$Score_{PATH3}$ gemäß dem Expressionsniveau von Genen der Nukleinsäuresequenzen SEQ ID NO:C1 bis SEQ ID NO:C6 berechnet wird und wobei k C und n 6 ist,
Die $Score_{PATH4}$ wird gemäß dem Expressionsniveau der Gene der Nukleinsäuresequenzen SEQ ID NO:D1 bis SEQ ID NO:D3 berechnet, wobei k gleich D und n gleich 3 ist, und
$Score_{PATH5}$ wird gemäß dem Expressionsniveau der Gene der Nukleinsäuresequenzen SEQ ID NO: E1 bis SEQ ID NO: E8 berechnet, wobei k gleich E und n gleich 8 ist;

c) wobei prognostiziert wird, dass:

i. wenn mindestens einer der $Score_{PATHJ}$ höher ist als ein entsprechender Referenzwert $Ref_J$, dann hat die Person wahrscheinlich ein schlechtes Ergebnis mit einer Ein-Jahres-Überlebensrate (OYS) unter 40 %,
ii. wenn alle $Score_{PATH1}$ bis $Score_{PATH5}$ niedriger oder gleich ihren jeweiligen Referenzwerten $Ref_1$ bis $Ref_5$ sind, dann hat die Person wahrscheinlich ein gutes Ergebnis mit einer OYS von über 50 %,
iii. Wenn der $Score_{PATH1}$ höher ist als sein jeweiliger Referenzwert $Ref_1$, dann ist es wahrscheinlich, dass die Person ein schlechtes Ergebnis mit einer OYS unter 20 % hat.

iv. Wenn der $Score_{PATH1}$ niedriger oder gleich seinem jeweiligen Referenzwert $Ref_1$ ist, dann ist es wahrscheinlich, dass die Person ein gutes Ergebnis mit einem OYS von über 55 % erzielt;

v. Wenn der $Score_{PATH2}$ höher ist als der entsprechende Referenzwert $Ref_2$, dann ist es wahrscheinlich, dass die Person ein schlechtes Ergebnis mit einem OYS unter 25 % erzielt;

vi. Wenn der $Score_{PATH2}$ niedriger oder gleich seinem jeweiligen Referenzwert $Ref_2$ ist, dann wird die Person wahrscheinlich ein gutes Ergebnis mit einem OYS von über 60 % erzielen.

vii. Wenn die $Score_{PATH3}$ höher ist als der jeweilige Referenzwert $Ref_3$, dann ist es wahrscheinlich, dass die Person ein schlechtes Ergebnis mit einem OYS unter 30 % erzielt;

viii. Wenn der $Score_{PATH3}$ niedriger oder gleich dem jeweiligen Referenzwert $Ref_3$ ist, dann ist es wahrscheinlich, dass die Person ein gutes Ergebnis mit einem OYS von über 70 % erzielt;

ix. Wenn der $Score_{PATH4}$ höher ist als der entsprechende Referenzwert $Ref_4$, dann ist es wahrscheinlich, dass die Person ein schlechtes Ergebnis mit einem OYS unter 10 % erzielt;

x. Wenn der $Score_{PATH4}$ niedriger oder gleich seinem jeweiligen Referenzwert $Ref_4$ ist, dann ist es wahrscheinlich, dass die Person ein gutes Ergebnis mit einem OYS von über 50 % erzielt;

xi. Wenn der $Score_{PATH5}$ höher als der entsprechende Referenzwert $Ref_5$ ist, ist es wahrscheinlich, dass die Person ein schlechtes Ergebnis mit einem OYS unter 40 % erzielt;

xii. Wenn der $Score_{PATH5}$ niedriger oder gleich seinem jeweiligen Referenzwert $Ref_5$ ist, dann ist es wahrscheinlich, dass die Person ein gutes Ergebnis mit einem OYS von über 80 % erzielt;

wobei der Referenzwert für $Score_{PATH1}$ ist $Ref_1$ und gleich 0,022276 ist,
der Referenzwert für $Score_{PATH2}$ ist $Ref_2$ und entspricht -0,57621,
der Referenzwert für $Score_{PATH3}$ ist $Ref_3$ und entspricht -0,26745 der Referenzwert für $Score_{PATH4}$ ist $Ref_4$ und entspricht 0,012234, und
der Referenzwert für $Score_{PATH5}$ ist $Ref_5$ und entspricht -1,1213.

2. Verfahren nach Anspruch 1, wobei Schritt c) ferner die folgende Prognose umfasst

xiii. Wenn beide $Score_{PATH3}$ und $Score_{PATH5}$ niedriger sind als ihre jeweiligen Referenzwerte $Ref_3$ und $Ref_5$, gehört die Person zu einer Gruppe mit geringem Risiko und einem OYS von über 80 %.

xiv. Wenn einer der $Score_{PATH3}$ und $Score_{PATH5}$ höher als sein jeweiliger Referenzwert $Ref_3$ oder $Ref_5$ ist und der andere niedriger als sein jeweiliger Referenzwert $Ref_3$ oder $Ref_5$, gehört die Person zu einer mittleren Risikogruppe mit einem OYS zwischen 60 und 80 %.

xv. Wenn sowohl $Score_{PATH3}$ als auch $Score_{PATH5}$ höher als ihre jeweiligen Referenzwerte $Ref_3$ und $Ref_5$ sind, gehört die Person zu einer Hochrisikogruppe mit einem OYS unter 30 %.

3. Verfahren nach Anspruch 2, wobei das Verfahren ferner die folgenden Schritte umfasst

d) Bestimmen des Mutationsstatus sowohl des NPM1-Gens als auch des FLT3-Gens, wobei das NPM1-Gen eine Nukleinsäure Sequenz gemäß SEQ ID NO:24 und das *FLT3*-Gen eine Nukleinsäure Sequenz gemäß SEQ ID NO:25 aufweist,

wobei, wenn das *NPM1-Gen* so mutiert ist, dass es für ein NPM1-Protein kodiert, das in das Zytoplasma delokalisiert ist, die Person als *NPM1+* klassifiziert wird,
andernfalls wird die Person als NPM1- klassifiziert,
wenn das *FLT3*-Gen so mutiert ist, dass es für ein FLT3-Protein mit einer internen Tandemduplikation in der Juxta-Transmembrandomäne kodiert, wird die Person als FLT3-ITD+ klassifiziert,
andernfalls wird die Person als FLT3-ITD- klassifiziert;

e) Klassifizierung der Person wie folgt:

**i.** Die Person gehört zu einer Gruppe A mit einem OYS von mehr als 85 %, wenn

- die Person *NPM1+* und FLT3-ITD- ist und gemäß Schritt c) zur Gruppe mit geringem Risiko oder mittlerem Risikogruppe gehört, oder
- die Person als *NPM1+* und FLT3-ITD+ oder NPM1- und FLT3-ITD-klassifiziert ist und gemäß Schritt c) zur Gruppe mit geringem Risikogruppe gehört

**ii.** die Person gehört zu einer Gruppe B mit einem OYS zwischen 40 und 60 %, wenn

- die Person als *NPM1+* und FLT3-ITD- klassifiziert ist und gemäß Schritt c) zur Hochrisikogruppe gehört,
- die Person als *NPM1+* und FLT3-ITD+ oder NPM1- und FLT3-ITD-klassifiziert ist und gemäß Schritt c) zur mittleren Risikogruppe gehört, oder
- die Person als NPM1- und FLT3-ITD+ klassifiziert ist und gemäß Schritt c) zur niedrigem Risikogruppe gehört

**iii.** die Person gehört zu einer Gruppe C mit einem OYS unter 30 %, wenn

- die Person als *NPM1+* und FLT3-ITD+ oder NPM1- und FLT3-ITD-klassifiziert ist und gemäß Schritt c) zur Hochrisikogruppe gehört, oder
- die Person als NPM1- und FLT3-ITD+ klassifiziert ist und gemäß Schritt c) zur mittleren Risikogruppe oder zur Hochrisikogruppe gehört.

**4.** Verfahren nach einem der Ansprüche 1 bis 3, wobei die biologische Probe aus der Gruppe ausgewählt wird, die eine Zellkultur, eine Zelllinie, eine Gewebebiopsie wie eine Knochenmarkaspiration, eine biologische Flüssigkeit wie Blut, Pleuraerguss und eine Serumprobe umfasst.

**5.** Verfahren nach einem der Ansprüche 1 bis 4, wobei das mindestens eine zur Behandlung von akuter myeloischer Leukämie verwendete Medikament aus Daunorubicin, Idarubicin, Cytarabin, Midostaurin, Cladribin, Gemtuzumab Ozogamicin oder einer Kombination davon ausgewählt ist.

**Revendications**

**1.** Procédé pour le pronostic *in vitro* du devenir d'un individu atteint d'une leucémie myéloïde aiguë (AML) sans anomalies chromosomiques et traité par au moins un médicament anti-AML, ledit procédé comprenant les étapes suivantes :

a) mesurer, dans un échantillon biologique provenant dudit individu, le niveau d'expression de l'ensemble des 23 gènes constitués des séquences d'acide nucléique telles que décrites dans SEQ ID NO:1 à SEQ ID NO:23 ;
b) calculer les scores J selon la formule suivante

$$Score_{PATHJ} = \sum_{i=k1}^{kn} \beta i \times Ci$$

où $\beta i$ représente la valeur de référence du coefficient de régression $\beta$ pour le gène de séquence d'acide nucléique SEQ ID NO:i,
où $Ci = 1$ si le niveau d'expression du gène de séquence d'acide nucléique SEQ ID NO:i est supérieur à un niveau d'expression de référence $ELR_i$ ou $Ci = -1$ si le niveau d'expression du gène de séquence d'acide nucléique SEQ ID NO:i est inférieur ou égal à $ELR_i$,
où J est un nombre entier compris entre 1 et 5 définissant cinq scores $Score_{PATH1}$ à $Score_{PATH5}$, où $Score_{PATH1}$ est calculé en fonction du niveau d'expression des gènes de séquences d'acide nucléique SEQ ID NO:A1 à SEQ ID NO:A4 et où k est A et n est 4,
$Score_{PATH2}$ est calculé en fonction du niveau d'expression des gènes de séquences d'acide nucléique SEQ ID NO:B1 à SEQ ID NO:B6 et où k est B et n est 6,
$Score_{PATH3}$ est calculé en fonction du niveau d'expression des gènes de séquences d'acide nucléique SEQ ID NO:C1 à SEQ ID NO:C6 et où k est C et n est 6,
$Score_{PATH4}$ est calculé en fonction du niveau d'expression des gènes de séquences d'acide nucléique SEQ ID NO:D1 à SEQ ID NO:D3, où k est D et n est 3, et
$Score_{PATH5}$ est calculé en fonction du niveau d'expression des gènes de séquences d'acide nucléique SEQ ID NO:E1 à SEQ ID NO:E8, où k est E et n est 8 ;

c) prédisant que :

i. si au moins un des scores $Score_{PATHJ}$ est supérieur à une valeur de référence respective $Ref_J$, alors l'individu est susceptible d'avoir un mauvais pronostic avec une survie à un an (OYS) inférieure à 40 %,

ii. si tous les scores Score$_{PATH1}$ à Score$_{PATH5}$ sont inférieurs ou égaux à leur valeur de référence respective Ref$_1$ à Ref$_5$, alors l'individu est susceptible d'avoir un bon pronostic avec une OYS supérieure à 50 %,

iii. si le score Score$_{PATH1}$ est supérieur à sa valeur de référence respective Ref$_1$, alors l'individu est susceptible d'avoir un mauvais pronostic avec une OYS inférieure à 20 %,

iv. si le score Score$_{PATH1}$ est inférieur ou égal à sa valeur de référence respective Ref$_1$, alors l'individu est susceptible d'avoir un bon pronostic avec une OYS supérieure à 55 %,

v. si le score Score$_{PATH2}$ est supérieur à sa valeur de référence respective Ref$_2$, alors l'individu est susceptible d'avoir un mauvais pronostic avec une OYS inférieure à 25 %,

vi. si le score Score$_{PATH2}$ est inférieur ou égal à sa valeur de référence respective Ref$_2$, alors l'individu est susceptible d'obtenir un bon résultat avec une OYS supérieure à 60 %,

vii. si le score Score$_{PATH3}$ est supérieur à sa valeur de référence respective Ref$_3$, alors l'individu est susceptible d'avoir un mauvais pronostic avec une OYS inférieure à 30 %,

viii. si le score Score$_{PATH3}$ est inférieur ou égal à sa valeur de référence respective Ref$_3$, alors l'individu est susceptible d'avoir un bon pronostic avec une OYS supérieure à 70 %,

ix. si le score Score$_{PATH4}$ est supérieur à sa valeur de référence respective Ref$_4$, alors l'individu est susceptible d'avoir un mauvais pronostic avec une OYS inférieure à 10 %,

x. si le score Score$_{PATH4}$ est inférieur ou égal à sa valeur de référence respective Ref$_4$, alors l'individu est susceptible d'avoir un bon pronostic avec une OYS supérieure à 50 %,

xi. si le score Score$_{PATH5}$ est supérieur à sa valeur de référence respective Ref$_5$, alors l'individu est susceptible d'avoir un mauvais pronostic avec une OYS inférieure à 40 %,

xii. si le score Score$_{PATH5}$ est inférieur ou égal à sa valeur de référence respective Ref$_5$, alors l'individu est susceptible d'avoir un bon pronostic avec une OYS supérieure à 80 %,

où la valeur de référence pour Score$_{PATH1}$ est Ref$_1$ et égale à 0,022276,

la valeur de référence pour Score$_{PATH2}$ est Ref$_2$ et égale à -0,57621,

la valeur de référence pour Score$_{PATH3}$ est Ref$_3$ et égale à -0,26745,

la valeur de référence pour Score$_{PATH4}$ est Ref$_4$ et égale à 0,012234, et

la valeur de référence pour Score$_{PATH5}$ est Ref$_5$ et égale à -1,1213.

**2.** Procédé selon la revendication 1, dans lequel l'étape c) comprend en outre le pronostic suivant

xiii. si les deux scores Score$_{PATH3}$ et Score$_{PATH5}$ sont inférieurs à leurs valeurs de référence respectives Ref$_3$ et Ref$_5$, alors l'individu appartient à un groupe à faible risque avec une OYS supérieure à 80 %,

xiv. si l'un des scores Score$_{PATH3}$ et Score$_{PATH5}$ est supérieur à sa valeur de référence respective Ref$_3$ ou Ref$_5$, et l'autre inférieur à sa valeur de référence respective Ref$_3$ ou Ref$_5$, alors l'individu appartient à un groupe à risque médian avec une OYS comprise entre 60 et 80 %,

xv. si Score$_{PATH3}$ et Score$_{PATH5}$ sont tous deux supérieurs à leurs valeurs de référence respectives Ref$_3$ et Ref$_5$, alors l'individu appartient à un groupe à risque élevé avec une OYS inférieure à 30 %.

**3.** Procédé selon la revendication 2, où le procédé comprend en outre les étapes suivantes étapes

d) déterminer le statut mutationnel des gènes *NPM1* et *FLT3,* ledit gène *NPM1* ayant une séquence d'acide nucléique telle que décrite dans SEQ ID NO:24 et ledit gène *FLT3* ayant une séquence d'acide nucléique telle que décrite dans SEQ ID NO:25, où

si le gène *NPM1* est muté de telle sorte qu'il code pour une protéine NMP1 qui est délocalisée dans le cytoplasme, l'individu est classé comme *NPM1+,*

sinon, l'individu est classé comme *NPM1-,*

si le gène *FLT3* est muté de telle sorte qu'il code pour une protéine FLT3 présentant une duplication interne en tandem dans le domaine juxta-transmembranaire, l'individu est classé comme *FLT3*-ITD+,

sinon l'individu est classé comme *FLT3*-ITD- ;

e) classer l'individu de telle sorte que

i. l'individu appartient à un groupe A avec une OYS supérieure à 85 % si

- l'individu est *NPM1+* et *FLT3*-ITD- et appartient au groupe à faible risque ou au groupe à risque médian selon l'étape c), ou
- l'individu est classé *NPM1+* et *FLT3*-ITD+ ou *NPM1-* et *FLT3*-ITD-, et appartient au groupe à faible risque selon l'étape c)

ii. la personne appartient à un groupe B avec une OYS comprise entre 40 et 60 %, si

- l'individu est classé *NPM1*+ et *FLT3*-ITD- et appartient au groupe à haut risque selon l'étape c),
- la personne est classée *NPM1*+ et *FLT3*-ITD+ ou *NPM1*- et *FLT3*-ITD-, et appartient au groupe à risque médian selon l'étape c), ou
- l'individu est classé *NPM1*- et *FLT3*-ITD+ et appartient au groupe à faible risque selon l'étape c)

iii. l'individu appartient à un groupe C avec une OYS inférieure à 30 % si

- l'individu est classé *NPM1*+ et *FLT3*-ITD+ ou *NPM1*- et *FLT3*-ITD-, et appartient au groupe à haut risque selon l'étape c), ou
- l'individu est classé *NPM1*- et *FLT3*-ITD+ et appartient au groupe à risque médian ou au groupe à haut risque selon l'étape c).

4. Procédé selon l'une quelconque des revendications 1 à 3, où l'échantillon biologique est sélectionné dans le groupe comprenant une culture cellulaire, une lignée cellulaire, une biopsie tissulaire telle qu'un prélèvement de moelle osseuse, un fluide biologique tel que du sang, un épanchement pleural et un échantillon de sérum.

5. Procédé selon l'une quelconque des revendications 1 à 4, dans lequel le au moins un médicament utilisé pour traiter la leucémie myéloïde aiguë est choisi parmi la daunorubicine, l'idarubicine, la cytarabine, la midostaurine, la cladribine, le gemtuzumab ozogamicine et une combinaison de ceux-ci.

Figure 1

Figure 2

Figure 3

Figure 4

Figure 5

Figure 6

30

Figure 7

Figure 8

**Figure 9**

**Figure 10**

| A | | | | |
|---|---|---|---|---|
| A | 27 | 25 | 17 | 12 | 1 |
| B | 32 | 16 | 11 | 6 | 1 |
| C | 19 | 5 | 2 | 2 | 0 |

Figure 11

| 1 | 38 | 25 | 20 | 15 |
| 2 | 95 | 33 | 22 | 15 |
| 3 | 29 | 8 | 4 | 1 |

Figure 12

Figure 13

Figure 14

Figure 15

Figure 16

Figure 17

Figure 18

Figure 19

Figure 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016012630 A
- WO 2013138237 A
- WO 2008066624 A
- US 7858331 B

**Non-patent literature cited in the description**

- **BRET et al.** *British Journal of Haematology*, 2015, vol. 169, 286-304
- **METZELER KH et al.** *Blood*, 2008, vol. 112, 4193-201